(19) Europäisches Patentamt
European Patent Office
Office européen des brevets

(11) **EP 1 205 473 A1**

(12) **EUROPEAN PATENT APPLICATION**
published in accordance with Art. 158(3) EPC

(43) Date of publication:
**15.05.2002 Bulletin 2002/20**

(21) Application number: **00948240.7**

(22) Date of filing: **25.07.2000**

(51) Int Cl.[7]: **C07D 295/096**, C07D 295/135, A61K 31/495, A61K 51/04

(86) International application number:
**PCT/JP00/04950**

(87) International publication number:
**WO 01/07427 (01.02.2001 Gazette 2001/05)**

(84) Designated Contracting States:
**AT BE CH CY DE DK ES FI FR GB GR IE IT LI LU MC NL PT SE**

(30) Priority: **28.07.1999 JP 21332199**

(71) Applicant: **DAIICHI RADIOISOTOPE LABORATORIES, LTD.**
**Tokyo 104-0031 (JP)**

(72) Inventors:
- **BANDO, Kazunori**
  **Sakura-shi, Chiba 285-0045 (JP)**
- **TAGUCHI, Kazumi**
  **Togane-shi, Chiba 283-0067 (JP)**
- **TAKATOKU, Keizo**
  **Inba-gun, Chiba 286-0201 (JP)**
- **NAGANUMA, Tomoyoshi**
  **Togane-shi, Chiba 283-0064 (JP)**
- **GINOZA, Yasushi**
  **Togane-shi, Chiba 283-0005 (JP)**
- **TANAKA, Yoshitomo**
  **Togane-shi, Chiba 283-0064 (JP)**

(74) Representative: **HOFFMANN - EITLE**
**Patent- und Rechtsanwälte**
**Arabellastrasse 4**
**81925 München (DE)**

(54) **VESAMICOL PIPERAZINE DERIVATIVES AND DRUGS CONTAINING THE SAME**

(57) A vesamicol piperazine derivative or a salt thereof of the following formula (I) is disclosed.

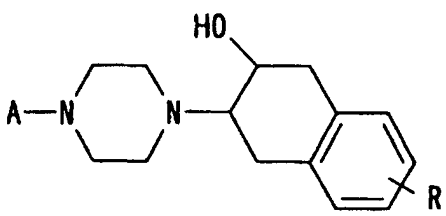

(I)

wherein R is a hydrogen atom, amino group, nitro group, non-radioactive or radioactive iodine atom, trialkyl tin group, or trialkyl silyl group, and A is a substituted or unsubstituted aryl group or a heterocyclic group.

The compound exhibits bonding properties precisely reflecting cholinergic neurons and high affinity to vesamicol receptors, and can specifically diagnose brain diseases, especially, Alzheimer's disease.

EP 1 205 473 A1

## Description

TECHNICAL FIELD

The present invention relates to a novel vesamicol piperazine derivative or a salt thereof and, more particularly, to a drug comprising the vesamicol piperazine derivative or the salt thereof as an effective component useful in the determination of central and peripheral acetylcholine transporters.

BACKGROUND ART

At present, there are about 1,300,000 dementia patients in Japan. This number is anticipated to increase in the future as the number of aged people rapidly increases. Senile dementia has previously been distinguished from cerebrovascular dementia as Alzheimer type dementia. More recently, however, as understanding of degenerative dementia becomes deeper, some types of degenerative dementia have been recognized as dementias independent from the Alzheimer type dementia. For this reason, development of an effective method of diagnosis which can exactly reflect the state of these diseases has been desired.

Alzheimer's disease is a disease most frequently encountered by persons suffering from senile dementia. Atrophy of the brain occurs in Alzheimer's disease, with the atrophy of the cerebral cortex being most significant. The atrophy is significant in the order of the frontal lobe, temporal lobe, parietal lobe, and occipital lobe. Brain atrophy is not specific to Alzheimer's disease, but can be experienced by elderly people between 60-100 who are clinically or pathologically normal. Atrophy of the frontal lobe and temporal lobe becomes remarkable as people become older. For this reason, early detection of Alzheimer's disease using a morphological diagnosis such as MRI and X-ray CT is difficult. In addition, it is impossible to detect lesions which can explain the patient's symptoms. Thus, the morphological diagnosis can only detect general brain atrophy of which the symptoms have considerably progressed.

An imaging method using a radioactive-labeled agent is known as a technique for cerebral function diagnostic imaging. Cerebral blood flow, glucose metabolism, oxygen metabolism, neurotransmitters, and the like can be directly measured and imaged by using this method. In research utilizing Positron Emission Tomography (PET) in which a positron emission nuclear species is used for the detection of Alzheimer's disease, degradation of forebrain glucose metabolism is seen according to the degree of dementia, or degradation of local oxygen metabolism or a decrease in the blood flow is experienced according to the type of dementia. The method of diagnosing Alzheimer's disease using PET, however, can be applied only in a very limited number of facilities in which a cyclotron for the production of a radioisotope (RI) is installed.

A decrease in the blood flow and a decrease in the oxygen metabolism or glucose metabolism are thought almost to coincide. Therefore, the cerebral blood flow determined by SPECT (Single Photon Emission Computed Tomography) using a single photon emission nuclear species is used for the diagnosis of Alzheimer's disease at the present time. The same diagnosis as can be done by the PET is possible today by using this method.

Although a decrease in the blood flow in Alzheimer's disease is seen in the temporoparietal association area and the posterior cingulated gyrus, the patterns are not necessarily the same. There are some varieties in the types, including a type in which the blood flow decreases only in the parietal lobe and temporal lobe, a type in which the blood flow decreases in all of the frontal lobe, parietal lobe, and temporal lobe, a type in which the decrease of the blood flow is predominant in the left side, and a type in which the decrease of the blood flow is predominant in the right side. This is caused by the change of the blood flow rate according to the severity of Alzheimer's disease and the use of a radioactive labeling agent nonspecific to Alzheimer's disease. Accumulation of such an agent is caused by a mechanism such as nonspecific bonding of the agent with proteins, decomposition of the agent by a nonspecific esterase, a structural change in the agent, and the like. Development of a method of diagnosing Alzheimer's disease which can ensure secure, specific, and early detection of Alzheimer's disease, therefore, will greatly contribute to curing and rehabilitation of Alzheimer's disease.

Abnormality of many neurotransmitters such as dopamine, serotonin, and noradrenalin in the brains of dead Alzheimer's disease patients has been reported. Among the neurotransmitters, cholinergic nerve transmitters exhibit most remarkable abnormality. Acetylcholine nerves have attracted attention due to the good correlation between the degree of disorder in the cholinergic system afferent fibers in the cerebral cortex and the degree of dementia, and also due to a decrease in the memory and learning capability of animals and humans having a disorder in the cholinergic system. In recent years, drugs capable of increasing an acetylcholine concentration in the choline nerve system have attracted attention as Alzheimer's disease curing drugs. Wide acceptance of this type of drugs in the future is expected also in Japan.

Acetylcholine is synthesized from choline and acetyl CoA by choline acetyltransferase which is an acetylcholine synthetase. Acetylcholine synthesized by the acetylcholine synthetase is transported to synaptic vesicles by an acetylcholine transporter and released therefrom in response to a stimulus to the nerves. The acetylcholine transporter

is specific to the acetylcholine nerve. Recent cloning research has clarified that a vesicle acetylcholine transporter is present in the first intron which is localized in a choline acetyltransferase gene (Eiden, J. Neurochem., 1998, 70, 2227-2240). This suggests that the choline acetyltransferase closely relates to the acetylcholine transporter and that the acetylcholine transporter as well as the choline acetyltransferase decreases with the progress of Alzheimer's disease.

As a drug which combines with the acetylcholine transporter, 2-(4-phenyl-piperidino)cyclohexanol (vesamicol), a compound having the following formula in which R is a hydrogen atom has been reported (Marshall, Br. J. Phamac., 1970, 38, 503-516). This compound was labeled with tritium and used as a ligand to detect the function of presynapse. Vesamicol, however, bonds with a sigma receptor (Custers et al, Eur. J. Pharmacol., 1997, 338, 177-183) or a "vesamicol bonding protein" (Hicks at al, J. Neurochem., 1991, 57, 509-591) which is not cholinergic neurons, but does not exhibit binding precisely reflecting binding to cholinergic neurons.
Furthermore, the affinity to the vesamicol receptor is not sufficiently high. m-Iodine vesamicol (mIV; a compound having an iodine atom for R in the following formula) with radioactive iodine directly introduced in vesamicol has been reported (Life Science 59, 1039-1045, 1996). The activity of mIV, however, is too small for the *in vivo* determination of cholinergic neurons.

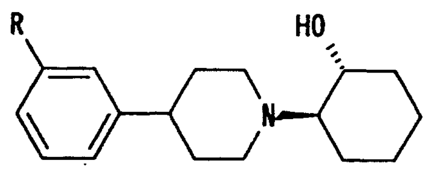

A variety of studies have been undertaken to improve low activity and specificity of the above vesamicol and mIV. For example, compounds such as a benzovesamicol derivative with a benzene ring attached to the vesamicol skeleton, 5-iodobenzovesamicol (IBVM) having an iodine atom for R in the following structure (Jung et al., J. Med. Chem. 33, 2065-2068, 1990), MABV having a methylamino group for R in the following structure (Mulholland and Jung, J. Labelled Comp. Radiopharm., 31, 253-259, 1992), and FEOBV having a 2-fluoroethyloxy group for R in the following structure (Mulholland's, J. Labelled Comp. Radiopharm., 33, 583-591, 1993) have been reported.

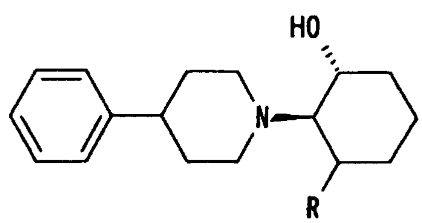

In addition, as asymmetric dipiperidine derivatives, a compound (MIBT) having a hydrogen atom for R and an iodine atom for R' in the following formula (Efange et al., J. Med. Chem., 36, 1754-1760, 1993), a compound (FBT) having a fluorine atom for R and a hydrogen atom for R' in the following formula (Efange et al., Appl. Radiat. Isot 45, 465-472, 1994), a comount (4-HIPP) with a structure obtainable by decyclizing a cyclohexanol (Efange et al., Nucl. Med. Biol. 19, 337-348, 1992), and the like have been reported.

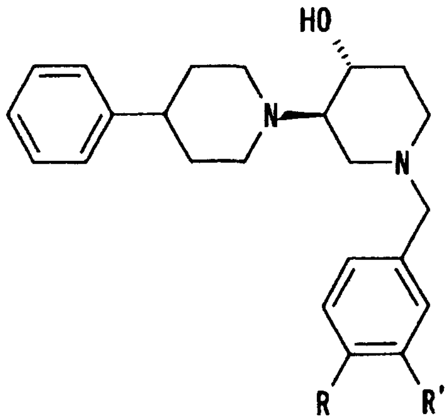

The compound first reported as an agent for Single Photon Emission Computed Tomography (SPECT) is 5-iodobenzovesamicol (IBVM) . A finding that the activity of vesamicol is increased by adding a benzene ring has been reported by G. A. Roger et al. at the University of California (J. Med. Chem., 32, 1217-1230, 1988). 5-iodobenzovesamicol was prepared by labeling this compound with radioactive iodine based on this finding. Although this compound has very high activity, our research is revealing that the compound bonds nonspecifically to positions other than acetylcholine nerves due to the high lipophilicity, thereby requiring a long period of time to obtain a contrast enabling diagnosis (Kuhl et al., J. Nucl. Med., 35, 405-410, 1994) and exhibits strong affinity to an $\alpha$1-receptor which is a receptor for adrenaline-type nerves ($IC_{50}$=6.74 nM).

With regard to asymmetric dipyridine derivatives, S. M. N. Efange et al. at the University of Minnesota have reported m-(iodobenzyl)trozamicol (MIBT) and
m-(iodobenzyl)spirotrozamicol having a spiro structure (Spiro MIBT, R=H, R'=I in the following formula).
2-Hydroxy-3-(4-iodinephenyl)-1-(4-phenyl-piperidinyl)propan e (4-HIPP) with a structure obtainable by decyclization of cyclohexanol has also been reported. These derivatives have affinity lower than 5-iodobenzovesamicol and have a problem of specificity. Their particularly high affinity to sigma receptors has been reported (Custers et al, Eur. J. Pharmacol., 338, 177-183, 1997).

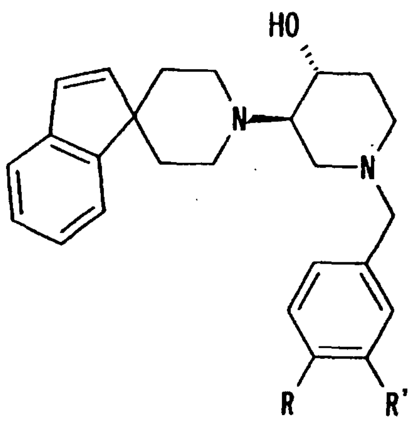

Development of a compound which exhibits binding properties precisely reflecting cholinergic neurons and high affinity to vesamicol receptors, as well as a technology capable of specifically diagnosing brain diseases, especially,
Alzheimer's disease by using such a compound, has been strongly desired. The present invention provides such a technology.

DISCLOSURE OF THE INVENTION

To solve various problems in the conventional vesamicol derivatives, particularly the problem of specificity of these derivatives, the inventors of the present invention have synthesized a number of new vesamicol derivatives and investigated their properties. As a result, the inventors have found that the specificity is greatly improved by changing the piperidine ring of benzovesamicol into a piperazine ring. For example, the compound DRC140 obtained by changing the piperidine ring of IBVM to a piperazine ring has an $IC_{50}$ value of 2.12 nM for vesamicol receptors, but an $IC_{50}$ value of 402 nM for $\alpha$1-receptor, which is an adrenaline-type nerve system receptor, indicating 190 times greater affinity to vesamicol receptors. The above compound also exhibited an activity to the vesamicol receptor 4,000 times as high as the activity to the sigma receptor having special problems relative to vesamicol and asymmetric dipiperidine derivatives. In this manner, the inventors have found that the specificity can be greatly improved by changing the piperidine ring of benzovesamicol into a piperazine ring.

Based on this finding, the present inventors have synthesized a number of derivatives having a basic skeleton in which the piperidine ring of benzovesamicol has been replaced by a piperazine ring to examine the activity of such derivatives to vesamicol receptors. As a result, the inventors have found that these compounds possess greatly increased activities to vesamicol receptors and can be used as radioactive drugs with excellent specificity capable of determining the amount of acetylcholine. This finding has led to the completion of the present invention.

Specifically, the present invention provides a vesamicol piperazine derivative or a salt thereof of the following formula:

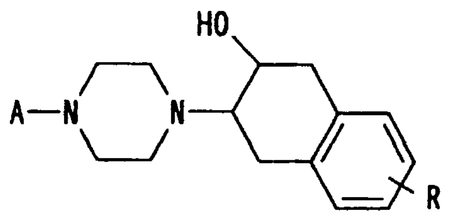

(I)

wherein R is a hydrogen atom, amino group, nitro group, non-radioactive or radioactive iodine atom, trialkyl tin group, or trialkyl silyl group, and A is a substituted or unsubstituted aryl group or a heterocyclic group.

The present invention also provides a drug comprising the above compound as an effective component useful for the determination of centeral and peripheral acetylcholine transporters.

BRIEF DESCRIPTION OF THE DRAWINGS

Figure 1 is a graph showing accumulation of a radioactive iodine-labeled DRE350 in various brain organizations of a rat, and Figure 2 shows autoradiography photographs of brain sections of the rat after administration of radioactive iodine-labeled DRE350.

BEST MODE FOR CARRYING OUT THE INVENTION

The vesamicol piperazine derivative of the present invention has a structure of the above-mentioned formula (I).

In the formula (I), as the trialkyltin group represented by R, a trimethyltin group, tributyltin group, and the like can be given. As the trialkylsilyl group, a trimethylsilyl group and the like can be given. As the radioactive iodine atom represented by the group R, $^{123}I$, $^{125}I$, $^{131}I$, and the like can be given, with $^{123}I$ being particularly preferable.

As examples of the group A in the formula (I), aryl groups such as a phenyl group, tolyl group, and xylyl group, and heterocyclic groups such as a pyridyl group and pyrimidyl group can be given. As examples of substituents for the group A, one or more of lower alkyl groups, amino groups, aminoalkyl groups, alkylamide groups, hydroxyl groups, hydroxyalkyl groups, lower alkoxy groups, non-radioactive or radioactive iodine atoms, trialkyltin groups, and trialkylsilyl groups can be given.

As examples of the lower alkyl groups among substituents for the group A, a methyl group, ethyl group, and the like can be given. The same groups as previously given in connection with radioactive iodine atoms, trialkyltin groups, and trialkylsilyl groups apply here. Of these substituents, particularly preferable groups are a methyl group and hydroxyl group.

Specific examples of the group A include 2-amino group, 2-aminomethyl group, 2-hydroxymethyl group, phenyl group, 2-methylphenyl group, 3-methylphenyl group, 2-ethoxyphenyl group, 4-methylphenyl group, 2,3-dimethylphenyl group, 2,5-dimethylphenyl group, 2,6-dimethylphenyl group, 2-methyl-5-methoxyphenyl group, 2-methyl-5-hydroxyphenyl group, 2-methyl-5-iodinephenyl group, 2-pyridyl group, and 2,6-pyrimidyl group.

The vesamicol piperazine derivatives (I) of the present invention have two asymmetric carbon atoms in the cyclohexanol ring. The two isomers are produced when the following processes are used for preparation. Specifically, two isomers, R,R-piperazine derivative ((-)-enantiomer) and S,S-piperazine derivative ((+)-enantiomer), are produced. Of these, the (-)-enantiomer is preferred.

The vesamicol piperazine derivatives (I) of the present invention can be prepared by any one of the following synthesis processes.

Synthesis process A

A piperazine compound of the formula (IV) is prepared by reacting an aniline compound of the formula (II) with bis(2-chloroethyl)amine hydrochloride of the formula (III). The piperazine compound (IV) is then reacted with N-(trifluoroacetyl)-1-amino-5,8-dihydronaphthalene oxide (V). The resulting reaction product is hydrolyzed to obtain a compound (Ia). If necessary, the amino group of the hydrolyzate (Ia) is converted into an iodine atom to obtain a compound (Ib).

(1)

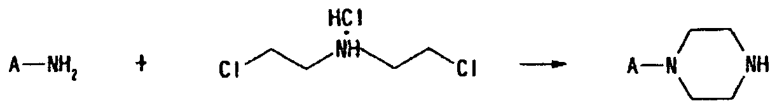

(II) (III) (IV)

(2)

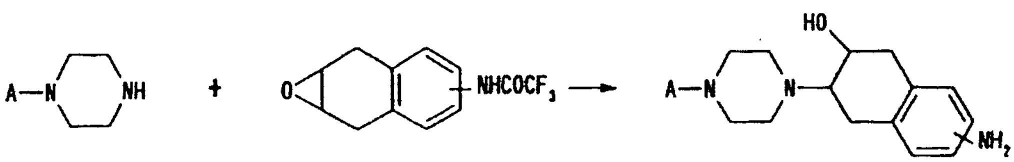

(IV) (V) (Ia)

(3)

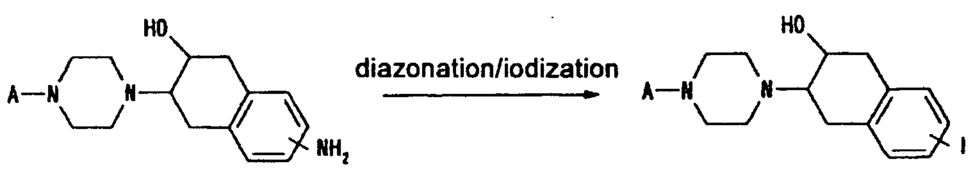


(Ia) (Ib)

The reaction of the aniline compound (II) and bis(2-chloroethyl)amine hydrochloride (III) is carried out by refluxing the mixture in the presence of a suitable solvent such as n-butanol, for example. The reaction of the piperazine compound (IV) and N-(trifluoroacetyl)-1-amino-5,8-dihydronaphthalene oxide (V) is also carried out by refluxing these compounds in a suitable solvent such as ethanol. Hydrolysis of the reaction product is carried out by causing a sodium hydroxide solution to react with the reaction product.

Conversion of the amino group in the hydrolyzate (Ia) into an iodine atom is carried out by diazonation of the

amino group with a nitrite in a mixed solvent of acetic acid and hydrochloric acid, followed by iodation of the resulting diazo compound using an iodation agent such as iodine and potassium iodide.

Although not shown in the above formula, a vesamicol piperazine derivative (I) having a trialkyltin group for the group R can be prepared by reacting a compound of the formula (Ib) with bistributyltin and tetrakistriphenylphosphine palladium, for example, in a suitable solvent such as toluene and replacing the iodine atom with a trialkyltin group. A vesamicol piperazine derivative (I) having a trialkylsilyl group for the group R can be prepared by reacting a compound of the formula (Ib) with bistributylsilane, for example.

Synthesis Process B

The piperazine compound (IV) prepared in Synthesis Process A is reacted with a dihydronaphthalene oxide derivative of the formula (VI). The resulting product is treated with an acid to obtain a compound of the formula (Ic). The amino group of this compound is removed to obtain a nitro compound (Id). Then, a compound (Ia) is obtained by converting the nitro group of the compound (Id) into an amino group. If required, a compound of the formula (Ib) is obtained by replacing the amino group of the compound (Ia) with an iodine atom.

(1)

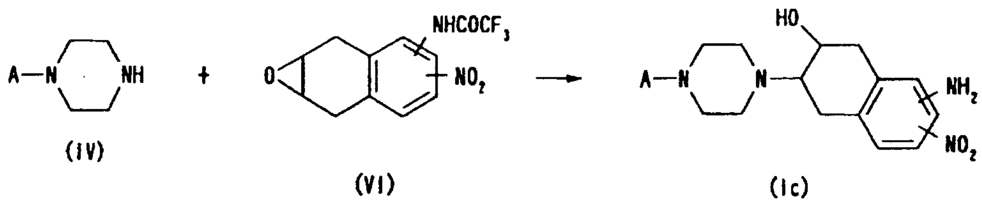


(2)

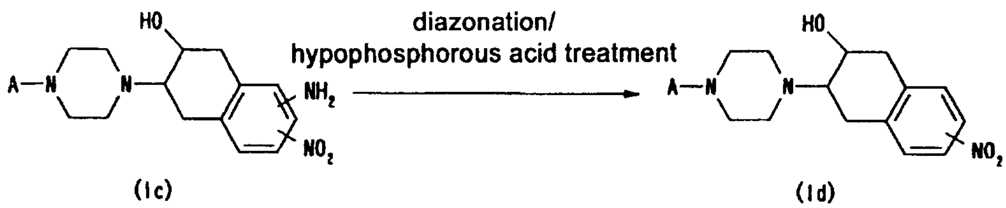


(3)

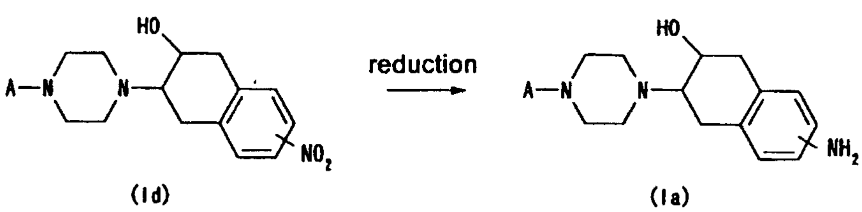

(4)

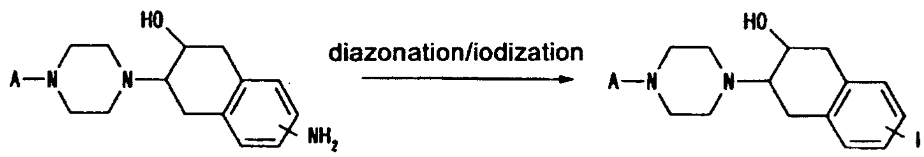


(Ia) (Ib)

The reaction of the piperazine compound (IV) and the dihydronaphthalene oxide derivative (VI) is carried out by refluxing the reaction mixture in the presence of a suitable solvent such as ethanol. The acid treatment of the resulting reaction product is carried out using a suitable mineral acid such as 6N hydrochloric acid. Removal of the amino group from the compound (Ic) is carried out by diazonating the amino group with a nitrite in a mixed solvent of acetic acid and hydrochloric acid, for example, followed by reaction with 50% hypophosphorous acid. To obtain the compound (Ia) by changing the nitro group of the compound (Id) into the amino group, a known nitro group reducing reaction can also be employed. A method of reduction using iron and hydrochloric acid in a mixed solvent of ethanol and water, for example, may be used. The method described above in the Synthesis Process A can be used for converting the amino group of the compound (Ia) to the iodine atom.

Synthesis process C

An N-benzylpiperazine dione compound of the formula (VIII) is prepared by reacting an aniline compound of the formula (II) with N-benzylimino diacetic acid of the formula (VII). The N-benzylpiperazine dione compound of the formula (VIII) is then reduced into N-benzyl piperazine compound of the formula (IX). A piperazine compound of the formula (IV) is obtained by catalytically reducing the N-benzyl piperazine compound of the formula (IX). The compound (Ib) is synthesized from the piperazine compound of the formula (IV) by a method described in the Synthesis Process A(2).

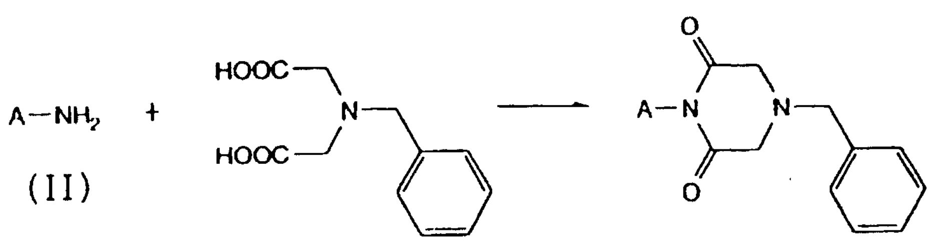

(II) (VII) (VIII)

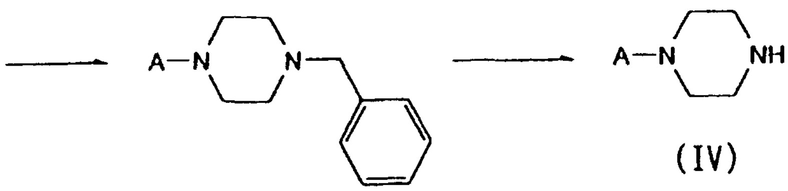

(IX)

(IV)

The reaction of the aniline compound (II) and N-benzylimino diacetic acid (VII) is carried out first by converting the N-benzylimino diacetic acid (VII) into an acid anhydride in the presence of a suitable acid anhydride such as acetic acid anhydride and the like, and then reacting the acid anhydride with the aniline compound (II). The reduction of the N-benzylpiperazine dione compound (VIII) is carried out in the presence of a suitable reducing agent such as boron hydride and the like. The catalytic reduction of N-benzylpiperazine compound (IX) is carried out in the presence of a suitable catalyst such as palladium/carbon.

Synthesis process D

According to the following formula, a naphthalene compound of the formula (X) is treated with an acid to remove an acetyl group, thereby obtaining a compound of the formula (XI) having no acetyl group. After removing the amino group from the naphthalene compound of the formula (XI), the resulting nitronaphthalene compound (XII) is treated with a peroxide to obtain an epoxy compound (XIII). The resulting epoxy compound is reacted with a compound of the formula (IV) to obtain a piperazine compound of the formula (Id). The compound (Ib) is synthesized from the piperazine compound by a method described in the Synthesis Process B(1) or B(2).

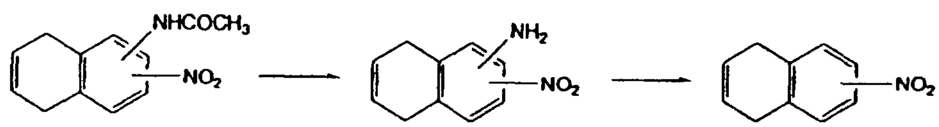

(X) (XI) (XII)

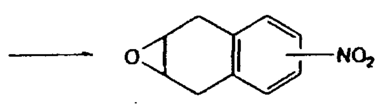

(XIII)

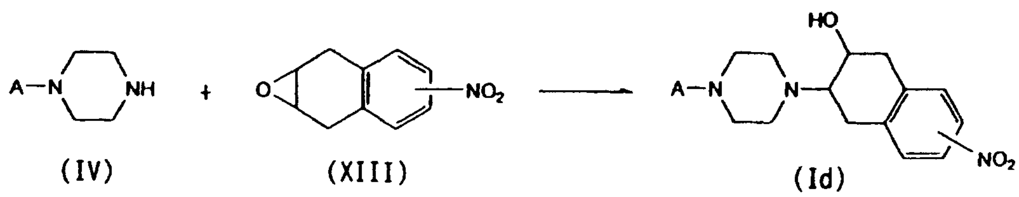

(IV) (XIII) (Id)

The acid treatment of the naphthalene compound (X) is carried out using a suitable mineral acid such as 6N hydrochloric acid, for example. Removal of the amino group from the compound (XI) is carried out by diazonating the amino group with a nitrite in a mixed solvent of acetic acid and hydrochloric acid, for example, followed by reaction with 50% hypophosphorous acid. Epoxidation of the nitronaphthalene compound (XII) is carried out using a suitable oxidizing agent such as 2-chloroperbenzoic acid, for example.

The vesamicol piperazine compound (I) prepared in this manner is an optically active substance and can be optically resolved. The method of optical resolution in the case of the compound (Ia), for example, is as follows.

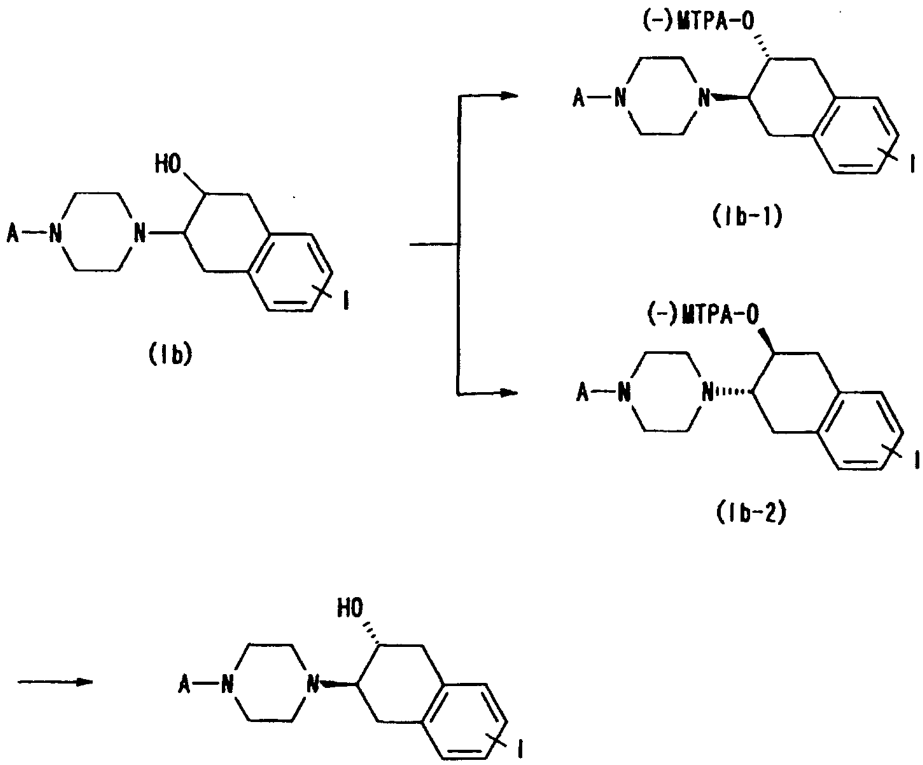

(Ib) (Ib-1) (Ib-2) (Ib-3)

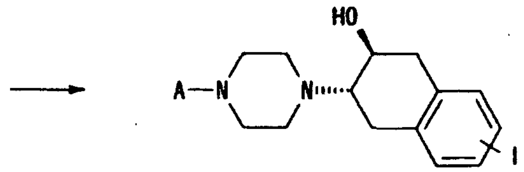

(Ib-4)

Specifically, after bonding the compound (Ia) with (-)-α-methoxy-α-trifluoromethylphenylacetyl chloride [(-) -MTPA-Cl], the compound is optically resolved and the (-) -MTPA is treated with an alkali to obtain (-) and (+) isomers of the the compound (Ia).

The vesamicol piperazine compound (I) obtained in this manner exhibits high specificity and strong affinity to vesamicol receptors as clearly shown in later-described Test Examples. Therefore, the vesamicol piperazine compound (I) labeled with radioactive iodine can be used as an agent for the determination of central and peripheral acetylcholine transformer porters. In addition, the compound is useful as a reagent for single photon emission computed tomography (SPECT).

To use the vesamicol piperazine compound (I) of the present invention, particularly the iodine labelled compound, as a drug or an agent, the compound obtained by the above-described method is purified, as required, by a known method, formulated with components which can be used as carriers for drugs, and prepared in any suitable dosing form.

As examples of drug carriers, carriers for oral or non-oral administration such as lactose, sucrose, glucose, starch, and crystalline cellulose can be given.

EXAMPLES

The present invention will be described in more detail by way of Examples and Test Examples which should not be construed as limiting the present invention.

Example 1

Synthesis of (±)-trans-5-iodo-2-hydroxy-3-[4-[2,5-dimethylphenyl]piperazinyl]tetralin (DRE348)

(1) Synthesis of (±)-trans-5-amino-2-hydroxy-3-[4-[2,5-dimethylphenyl]piperazinyl]tetralin

20 ml of ethanol was added to 740 mg (3.889 mmol) of 1-(2,5-dimethylphenyl)piperazine to dissolve the compund. 1.0 g (3.888 mmol) of N-[trifluoroacetyl]-1-amino-5,8-dihydronaphthalene oxide was added to the reaction solution and the mixture was refluxed for 22 hours. After cooling the reaction solution, the solvent was removed by evaporation and the residue was dissolved in 10 ml of methanol. 20 ml of 2N sodium hydroxide was added to the solution and the mixture was stirred for 20 hours. The reaction solution was extracted with a mixture of water/dichloromethane, the organic layer was dried over anhydrous sodium sulfate, and the solvent was evaporated. The residue was purified by flash chromatography (dichloromethane:methanol = 98:2) to obtain 548 mg (40.0%) of (±)-trans-5-amino-2-hydroxy-3-[4-[2,5-dimethylphenyl]-piperazinyl]tetralin as a light yellow amorphous low polar substance.
TLC (dichloromethane:methanol = 98:2); Rf = 0.45

(2) Synthesis of (±)-trans-5-iodo-2-hydroxy-3-[4-[2,5-dimethylphenyl]piperazinyl]tetralin (DRE348)

8 ml of acetic acid and 4 ml of concentrated hydrochloric acid were added to 548 mg (1.559 mmol) of (±)-trans-5-amino-2-hydroxy-3-[4-[2,5-dimethylphenyl]piperazinyl]tetralin and the mixture was stirred to cool with ice to a temperature of 5°C or less. A solution of 120 mg of sodium nitrite (1.739 mmol in 8 ml of water) was slowly added dropwise while maintaining the temperature of the reaction solution at 7°C or less. The reaction solution was stirred for 20 minutes while controlling the temperature at 5°C. Next, a solution of potassium iodide (320 mg(1.928 mmol)) and iodine (244 mg (0.961 mmol)) in 4 ml of water was slowly added dropwise while controlling the temperature at 7°C. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The residue was purified by chromatography (dichloromethane: methanol = 98:2) to obtain 461 mg (64.0%) of (±)-trans-5-iodo-2-hydroxy-3-[4-[2,5-dimethylphenyl] piperazinyl]tetralin as a brown amorphous substance. $^1$H-NMR($CDCl_3$)δ:

7.69(d,J=7.57Hz,1H), 6.76-7.28(m,5H), 3.74-3.99(m,1H),
2.49-3.37(m,13H), 2.30(s,6H)

TLC (dichloromethane:methanol = 97:3); Rf = 0.74
FAB/MS(m/z):463($MH^+$)

Example 2

Synthesis of (+)-trans-5-iodo-2-hydroxy-3-[4-[2,5-dimethylphenyl]piperazinyl]tetralin (DRE351) and (-) -trans-5-iodo-2-hydroxy-3-[4-[2,5-dimethylphenyl] piperazinyl]tetralin (DRE350)

(1) Synthesis of (+)-trans-5-iodo-2-α-methoxy-α-trifluoromethylphenylacetoxy-3-[4-[2,5-dimethylphenyl] piperazinyl] tetralin and (-)-trans-5-iodo-2-α-methoxy-α-trifluoromethylphenylacetoxy-3-[4-[2,5-dimethylphenyl] piperazinyl]tetralin

324 mg (0.701 mmol) of (±)-trans-5-iodo-2-hydroxy-3-[4-[2,5-dimethylphenyl]piperazinyl]tetralin was dissolved in 7.2 ml of dichloromethane and 2 mg (0.016 mmol) of 4-dimethylamino-pyridine (DMAP) and 97 μl (0.700 mmol) of triethylamine were added. The mixture was cooled to -80°C with stirring: 200 mg (0.792 mmol) of (-)-α-methoxy-α-trifluoromethylphenylacetylchloride ((-)-MTPA-Cl) was slowly added to the reaction solution. The reaction mixture was stirred for 30 minutes at -80°C and kept 24 hours at room temperature. The reaction was terminated by adding water and the reaction solution was extracted with a mixture of water and dichloromethane. The organic layer was dried over anhydrous sodium sulfate and the solvent was evaporated under vacuum. The residue was purified by flash chromatography (dichloromethane) and the solvent was evaporated to obtain 139 mg (54.8%) of (+)-trans-5-iodo-2-α-methoxy-α-trifluoromethylphenylacetoxy-3-[4-[2,5-dimethylphenyl] piperazinyl]tetralin and 90 mg (37.8%) of (-)-trans-5-iodo-2-α-methoxy-α-trifluoromethylphenylacetoxy-3-[4-[2,5-dimethylphenyl] piperazinyl]tetralin.

· (+)-trans-5-iodo-2-α-methoxy-α-trifluoromethyl-phenylacetoxy -3-[4-[2,5-dimethylphenyl]piperazinyl]tetralin
TLC (dichloromethane:n-hexane = 7:3); Rf = 0.60
$[\alpha]^{23}_D$ = +13.2 (C = 1.0, chloroform)
· (-)-trans-5-iodo-2-α-methoxy-α-trifluoromethylphenyl-acetoxy-3-[4-[2,5-dimethylphenyl]piperazinyl]tetralin
TLC (dichloromethane:n-hexane = 7:3); Rf = 0.47
$[\alpha]^{23}_D$ = -59.5 (C = 1.0, chloroform)

(2) Synthesis of (+)-trans-5-iodo-2-hydroxy-3-[4-[2,5-dimethylphenyl]piperazinyl]tetralin (DRE351)

139 mg (0.205 mmol) of (+)-trans-5-iodo-2-α-methoxy-α-trifluoromethylphenylacetoxy-3-[4-[2,5-dimethylphenyl]-piperazinyl]tetralin was dissolved in 2 ml of tetrahydrofuran. After the addition of 5 ml of 6N sodium hydroxide, the mixture was stirred for 72 hours at room temperature. The reaction solution was extracted with a mixture of water/dichloromethane, the organic layer was dried over anhydrous sodium sulfate, and the solvent was evaporated. The residue was purified by chromatography (dichloromethane → dichloromethane:methanol = 97:3) and the solvent was evaporated to obtain 83 mg (87.4%) of the title compound as a brown amorphous substance.
$[\alpha]^{23}_D$ = +35.8 (C = 1.0, chloroform)

(3) Synthesis of (-)-trans-5-iodo-2-hydroxy-3-[4-[2,5-dimethylphenyl]piperazinyl]tetralin (DRE350)

90 mg (0.133 mmol) of (-)-trans-5-iodo-2-α-methoxy-α-trifluoromethylphenylacetoxy-3-[4-(2,5-dimethylphenyl] piperazinyl]tetralin was dissolved in 2 ml of tetrahydrofuran. After the addition of 5 ml of 6N sodium hydroxide, the mixture was stirred for 72 hours at room temperature. The reaction solution was extracted with a mixture of water/dichloromethane, the organic layer was dried over anhydrous sodium sulfate, and the solvent was evaporated. The residue was purified by chromatography (dichloromethane → dichloromethane:methanol = 97:3) and the solvent was evaporated to obtain 57 mg (91.9%) of the title compound as a brown amorphous substance.
$[\alpha]^{23}_D$ = -59.0 (C = 1.0, chloroform)

Example 3

(+)-trans-5-tributylstannyl-2-hydroxy-3-[4-[2,5-dimethylphenyl]piperazinyl]tetralin (DRE364) and (-)-trans-5-tributylstannyl-2-hydroxy-3-[4-[2,5-dimethylphenyl]piperazinyl]tetralin (DRE363)

(1) Synthesis of (+)-trans-5-tributylstannyl-2-hydroxy-3-[4-[2,5-dimethylphenyl]piperazinyl]tetralin (DRE364)

73 mg (0.158 mmol) of (+)-trans-5-iodo-2-hydroxy-3-[4-[2,5-dimethylphenyl]piperazinyl]tetralin was dissolved in 2 ml of toluene and argon gas was bubbled through the mixture for 5 minutes to deaerate the mixture. After the addition of 170 µl (0.336 mmol) of bis(tributyltin) and 10 mg (0.009 mmol) of tetrakistriphenylphosphine palladium, the reaction solution was refluxed for 15 hours in an argon gas atmosphere. After removing the solvent by evaporation, the residue was purified by chromatography (dichloromethane → dichloromethane:ethyl acetate = 9:1) and NH chromatography (dichloromethane:ethyl acetate = 9:1). The solvent was evaporated to obtain 10 mg (10.1%) of the title compound as a yellowish brown amorphous substance.
TLC (dichloromethane:methanol = 98:2); Rf = 0.70
$[\alpha]^{23}_{D}$ = +17.0 (C = 1.0, chloroform)

(2) Synthesis of (-)-trans-5-tributylstannyl-2-hydroxy-3-[4-[2,5-dimethylphenyl]piperazinyl]tetralin (DRE363)

47 mg (0.102 mmol) of (-)-trans-5-iodo-2-hydroxy-3-[4-[2,5-dimethylphenyl]piperazinyl]tetralin was dissolved in 2 ml of toluene and argon gas was bubbled through the mixture for 5 minutes to deaerate the mixture. After the addition of 110 µl (0.218 mmol) of bis(tributyltin) and 7 mg (0.006 mmol) of tetrakistriphenylphosphine palladium, the reaction solution was refluxed for 17 hours in an argon gas atmosphere. After removing the solvent by evaporation, the residue was purified by chromatography (dichloromethane → dichloromethane:ethyl acetate = 9:1) and NH chromatography (dichloromethane:ethyl acetate = 9:1). The solvent was evaporated to obtain 28 mg (43.8%) of the title compound as a yellowish brown amorphous substance.
$^{1}$H-NMR($CDCl_3$)δ:
6.75-7.34(m,6H), 3.68-4.00(m,1H), 2.67-3.45(m,13H),
2.31(s,6H), 0.82-1.58(m,27H)
TLC (dichloromethane:methanol = 99:1); Rf = 0.60
$[\alpha]^{23}_{D}$ = -50.3 (C = 1.0, chloroform)
FAB/MS(m/z); 625(MH)

Example 4

Synthesis of (±)-trans-6-iodo-2-hydroxy-3-[4-[2,5-dimethylphenyl]piperazinyl]tetralin (DRE365) and (±)-trans-7-iodo-2-hydroxy-3-[4-[2,5-dimethylphenyl]-piperazinyl]tetralin (DRE366)

(1) Synthesis of (±)-trans-N-acetyl-5-amino-6-nitro-2-hydroxy-3-[4-[2,5-dimethylphenyl]piperazinyl]tetralin and (±)-trans-N-acetyl-8-amino-7-nitro-2-hydroxy-3-[4-[2,5- dimethylphenyl]piperazinyl]tetralin

3.3 g (13.249 mmol) of N-acetyl-1-amino-2-nitro-5,8-dihydronaphthalene oxide was dissolved in 45 ml of ethanol and 2.5 g (13.138 mmol) of 1-[2, 5-dimethylphenyl]piperazine was added. The mixture was refluxed for 19 hours. The reaction solution was cooled to room temperature and the solvent was evaporated. The residue was purified by chromatography (dichloromethane:ethyl acetate = 9:1 → dichloromethane:methanol = 97:3) and the solvent was evaporated to obtain 616 mg (10.7%) of (±)-trans-N-acetyl-5-amino-6-nitro-2-hydroxy-3-[4-[2,5-dimethylphenyl]piperazinyl] tetralin as a yellow amorphous substance and 612 mg (10.6%) of (±)-trans-N-acetyl-8-amino-7-nitro-2-hydroxy-3-[4-[2,5-dimethylphenyl]piperazinyl] tetralin as a yellow amorphous substance.

- (±)-trans-N-acetyl-5-amino-6-nitro-2-hydroxy-3-[4-[2,5-dimethylphenyl]piperazinyl]tetralin
  TLC (dichloromethane:methanol = 97:3); Rf = 0.61
  FAB/MS (m/z); 439($MH^+$)
- (±)-trans-N-acetyl-8-amino-7-nitro-2-hydroxy-3-[4-[2,5-dimethylphenyl]piperazinyl]tetralin
  TLC (dichloromethane:methanol = 97:3); Rf = 0.51
  FAB/MS (m/z) ; 439($MH^+$)

(2) Synthesis of (±)-trans-5-amino-6-nitro-2-hydroxy-3-[4-[2,5-dimethylphenyl]piperazinyl]tetralin

6N hydrochloric acid was added to 613 mg (1.394 mmol) of (±)-trans-N-acetyl-5-amino-6-nitro-2-hydroxy-3-[4-[2,5-dimethylphenyl]piperazinyl]tetralin and the mixture was refluxed for two hours. The reaction solution was alkalinized with 6N sodium hydroxide while cooling with ice. The solution was then extracted with a mixture of water/dichloromethane, the organic layer was dried over anhydrous sodium sulfate, and the solvent was evaporated. The residue was purified by chromatography (dichloromethane:methanol = 97:3) and the solvent was evaporated to obtain 515 mg (93.0%) of the title compound as a yellow amorphous substance.
TLC (dichloromethane:methanol = 97:3); Rf = 0.64

(3) Synthesis of (±)-trans-6-nitro-2-hydroxy-3-[4-[2,5-dimethylphenyl]piperazinyl]tetralin

8 ml of acetic acid and 4 ml of concentrated hydrochloric acid were added to 515 mg (1.299 mmol) of (±)-trans-5-amino-6-nitro-2-hydroxy-3-[4-[2,5-dimethyl- phenyl]piperazinyl] tetralin and the mixture was cooled with ice. 104 mg (1.507 mmol in 8 ml of water) of sodium nitrite was slowly added to the reaction solution while controlling the temperature at 7°C or less, following which the mixture was stirred for one hour at a temperature of at 5°C or less. 8 ml of 50% hypophosphorous acid was slowly added to the reaction solution, and the mixture was stirred overnight at room temperature. The reaction solution was alkalinized with the addition of 2N sodium hydroxide while stirring and cooling with ice. The reaction solution was extracted with a mixture of water/dichloromethane, the organic layer was dried over anhydrous sodium sulfate, and the solvent was evaporated. The residue was purified by chromatography (dichloromethane:methanol = 99:1) and the solvent was evaporated to obtain 339 mg (68.4%) of the title compound as a yellow amorphous substance.
TLC (dichloromethane:methanol = 99:1); Rf = 0.44

(4) Synthesis of (±)-trans-6-amino-2-hydroxy-3-[4-[2,5-dimethylphenyl]piperazinyl]tetralin

1.8 g of iron powder, 8 ml of 75% ethanol, and 500 μl of concentrated hydrochloric acid were added to 339 mg (0.889 mmol) of (±)-trans-6-nitro-2-hydroxy-3-[4-[2,5-dimethylphenyl]piperazinyl]tetralin and the mixture was refluxed for one hour. The reaction mixture was allowed to cool to room temperature and filtered through celite. The filtrate was alkalinized with the addition of 2N sodium hydroxide and extracted using a water/dichloromethane mixture. The organic layer was dried over anhydrous sodium sulfate and the solvent was evaporated. The residue was purified by chromatography (dichloromethane:methanol = 97:3) and the solvent was evaporated to obtain 225 mg (72.1%) of the title compound as a brown amorphous substance.
TLC (dichloromethane:methanol = 97:3); Rf = 0.36

(5) Synthesis of ((+)-trans-6-iodo-2-hydroxy-3-[4-[2,5-dimethylphenyl]piperazinyl]tetralin (DRE365)

4 ml of acetic acid and 2 ml of concentrated hydrochloric acid were added to 225 mg (0.640 mmol) of (±)-trans-6-amino-2-hydroxy-3-[4-[2,5-dimethylphenyl]piperazinyl]tetralin and the mixture was stirred to cool with ice to a temperature of 5°C or less. A solution of 51 mg of sodium nitrite (0.739 mmol in 4 ml of water) was slowly added dropwise while maintaining the temperature of the reaction solution at 7°C or less. The reaction solution was stirred for 20 minutes while controlling the temperature at 5°C. Next, a solution of potassium iodide (131 mg(0.789 mmol)) and iodine (100 mg (0.394 mmol)) in 2 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 99:1) to obtain 159 mg (53.7%) of the title compound as a brown amorphous substance.
$^1$H-NMR($CDCl_3$) δ:
6.76-7.59(m,6H), 3.65-3.98(m,1H), 2.58-3.59(m,13H),
2.30(s,3H), 2.27(s,3H)
TLC (dichloromethane:methanol = 97:3); Rf = 0.59
FAB/MS (m/z); 463($MH^+$)

(6) Synthesis of (±)-trans-8-amino-7-nitro-2-hydroxy-3-[4-[2,5-dimethylphenyl]piperazinyl]tetralin

20 ml of 6N hydrochloric acid was aded to 611 mg (1.393 mmol) of (±)-trans-N-acetyl-8-amino-7-nitro-2-hydroxy-3-[4-[2,5-dimethylphenyl]piperazinyl]tetralin and the mixture was refluxed for two hours. The reaction solution was alkalinized with 6N sodium hydroxide while cooling with ice. The solution was then extracted with a mixture of water/dichloromethane, the organic layer was dried over anhydrous sodium sulfate, and the solvent was evaporated. The residue was purified by chromatography (dichloromethane:methanol = 97:3) and the solvent was evaporated to obtain 375 mg (67.9%) of the title compound as a yellow amorphous substance.

TLC (dichloromethane:methanol = 97:3); Rf = 0.51

(7) Synthesis of (±)-trans-7-nitro-2-hydroxy-3-[4-[2,5-dimethylphenyl]piperazinyl]tetralin

6 ml of acetic acid and 3 ml of concentrated hydrochloric acid were added to 380 mg (0.958 mmol) of (±)-trans-8-amino-7-nitro-2-hydroxy-3-[4-[2,5-dimethylphenyl]piperazinyl] tetralin and the mixture was cooled with ice. 77 mg (1.116 mmol in 6 ml of water) of sodium nitrite was slowly added to the reaction solution while controlling the temperature at 7°C or less, following which the mixture was stirred for one hour at a temperature of at 5°C or less. 6 ml of 50% hypophosphorous acid was slowly added to the reaction solution, and the mixture was stirred overnight at room temperature. The reaction solution was alkalinized with the addition of 2N sodium hydroxide while stirring and cooling with ice. The reaction solution was extracted with a mixture of water and dichloromethane, the organic layer was dried over anhydrous sodium sulfate, and the solvent was evaporated. The residue was purified by chromatography (dichloromethane:methanol = 97:3) and the solvent was evaporated to obtain 237 mg (64.8%) of the title compound as a yellow amorphous substance.

TLC (dichloromethane:methanol = 97:3); Rf = 0.75

(8) Synthesis of (±)-trans-7-amino-2-hydroxy-3-[4-[2,5-dimethylphenyl]piperazinyl]tetralin

1.2 g of iron powder, 10 ml of 75% ethanol, and 500 µl of concentrated hydrochloric acid were added to 237 mg (0.621 mmol) of (±)-trans-7-nitro-2-hydroxy-3-[4-[2,5-dimethylphenyl]piperazinyl]tetralin and the mixture was refluxed for one hour. The reaction mixture was allowed to cool to room temperature and filtered through celite. The filtrate was alkalinized with the addition of 2N sodium hydroxide and extracted using a water/dichloromethane mixture. The organic layer was dried over anhydrous sodium sulfate and the solvent was evaporated. The residue was purified by chromatography (dichloromethane:methanol = 97:3) and the solvent was evaporated to obtain 164 mg (75.2%) of the title compound as a brown amorphous substance.

TLC (dichloromethane:methanol = 97:3); Rf = 0.30

(9) Synthesis of ((+)-trans-7-iodo-2-hydroxy-3-[4-[2,5-dimethylphenyl]piperazinyl]tetralin (DRE366)

2.6 ml of acetic acid and 1.3 ml of concentrated hydrochloric acid were added to 164 mg (0.467 mmol) of (±)-trans-7-amino-2-hydroxy-3-[4-[2,5-dimethylphenyl] piperazinyl]tetralin and the mixture was stirred to cool with ice to a temperature of 5°C or less. A solution of 35 mg of sodium nitrite (0.506 mmol in 2.6 ml of water) was slowly added dropwise while maintaining the temperature of the reaction solution at 7°C or less. The reaction solution was stirred for 20 minutes while controlling the temperature at 5°C. Next, a solution of potassium iodide (95 mg (0.572 mmol)) and iodine (71 mg (0.280 mmol)) in 1.3 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane: ethyl acetate = 9:1) to obtain 87 mg (40.3%) of the title compound as a brown amorphous substance.

$^1$H-NMR($CDCl_3$) δ:

6.78-7.60(m,6H), 3.74-3.95(m,1H), 2.60-3.40(m,13H),
2.31(s,3H), 2.28(s,3H)

TLC (dichloromethane: ethyl acetate = 8:2); Rf = 0.55
FAB/MS (m/z); 463($MH^+$)

Example 5

(±)-trans-5-iodo-2-hydroxy-3-[4-[2-methylphenyl]-piperazinyl]tetralin (DRE334) and (+)-trans-8-iodo-2-hydroxy-3-[4-[2-methylphenyl]piperazinyl]tetralin (DRE335)

(1) Synthesis of (±)-trans-5-amino-2-hydroxy-3-[4-[2-methylphenyl]piperazinyl]tetralin and (±)-trans-8-amino-2-hydroxy-3-[4-[2-methylphenyl]-piperazinyl]tetralin

40 ml of ethanol and 2.2 ml of triethylamine were added to 3.3 g (15.153 mmol) of 1-(2-tolyl)piperazine hydrochloride and dissolved by heating while stirring. 2.0 g (7.776 mmol) of N-[trifluoroacetyl]-1-amino-5,8-dihydronaphthalene oxide was added to the reaction solution and the mixture was refluxed for 20 hours. After cooling the reaction solution, the solvent was removed by evaporation and the residue was dissolved in 14 ml of methanol. 40 ml of 2N sodium hydroxide was added to the solution and the mixture was stirred for 20 hours . The reaction solution was extracted with a mixture of water and dichloromethane, the organic layer was dried over anhydrous sodium sulfate, and the solvent was evaporated. The residue was purified by flash chromatography (dichloromethane:methanol = 98:2) to obtain 1.19 g (45.4%) of (±)-trans-5-amino-2-hydroxy-3-[4-[2-methylphenyl] piperazinyl]tetralin as a white amorphous low polar substance and 0.97 g (37.0%) of (±)-trans-8-amino-2-hydroxy-3-[4-[2-methylphenyl]piperazinyl]tetralin as a pale brown amorphous high polar substance.

- (±)-trans-5-amino-2-hydroxy-3-[4-[2-methylphenyl]-piperazinyl]tetralin
  TLC (dichloromethane:methanol = 95:5); Rf = 0.76
- (±)-trans-8-amino-2-hydroxy-3-[4-[2-methylphenyl]-piperazinyl]tetralin
  TLC (dichloromethane:methanol = 95:5); Rf = 0.52

(2) Synthesis of (±)-trans-5-iodo-2-hydroxy-3-[4-[2-methylphenyl]piperazinyl]tetralin (DRE334)

15 ml of acetic acid and 7 ml of concentrated hydrochloric acid were added to 1.19 g (3.526 mmol) of (±)-trans-5-amino-2-hydroxy-3-[4-[2-methylphenyl]piperazinyl]tetralin and the mixture was stirred to cool with ice to a temperature of 5°C or less. A solution of 262 mg of sodium nitrite (3.797 mmol in 15 ml of water) was slowly added dropwise while maintaining the temperature of the reaction solution at 7°C or less. The reaction solution was stirred for 20 minutes while controlling the temperature at 5°C. Next, a solution of potassium iodide (704 mg (4.241 mmol)) and iodine (538 mg (2.12 mmol)) in 7 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 99:1) to obtain 1.4 g (87.5%) of the title compound as a reddish brown amorphous substance.

$^1$H-NMR($CDCl_3$) $\delta$:
7.70(d, J=7.80Hz,1H), 6.47-7.24(m,6H), 3.71-3.93(m,1H),
2.52-3.53(m,13H), 2.34(s,3H)
TLC (dichloromethane:methanol = 95:5); Rf = 0.8
FAB/MS(m/z); 449($MH^+$)

(3) Synthesis of (+)-trans-8-iodo-2-hydroxy-3-[4-[2-methylphenyl]piperazinyl]tetralin (DRE335)

6 ml of acetic acid and 3 ml of concentrated hydrochloric acid were added to 657 mg (1.946 mmol) of (±)-trans-8-amino-2-hydroxy-3-[4-[2-methylphenyl]piperazinyl]tetralin and the mixture was stirred to cool with ice to a temperature of 5°C or less. A solution of 145 mg of sodium nitrite (2.101 mmol in 6 ml of water) was slowly added dropwise while maintaining the temperature of the reaction solution at 7°C or less. The reaction solution was stirred for 20 minutes while controlling the temperature at 5°C. Next, a solution of potassium iodide (388 mg(2.337 mmol)) and iodine (296 mg (1.166 mmol)) in 3 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 99:1) to obtain 344 mg (39.5%) of the title compound as a light yellow amorphous substance.

$^1H$-NMR($CDCl_3$)δ:
7.69(d,J=7.82Hz,1H), 6.72-7.22(m,6H), 3.67-3.99(m,1H), 3.27-3.53(m,1H), 2.46-2.97(m,12H), 2.37(s,3H)
TLC (dichloromethane:methanol = 95:5); Rf = 0.83
FAB/MS (m/z) ; 449($MH^+$)

Example 6

(+)-trans-5-iodo-2-hydroxy-3-[4-[2-methylphenyl]-piperazinyl]tetralin (DRE341) and (-)-trans-5-iodo-2-hydroxy-3-[4-[2-methylphenyl]piperazinyl]tetralin (DRE342)

(1) Synthesis of (+)-trans-5-iodo-2-α-methoxy-α-trifluoromethylphenylacetoxy-3-[4-[2-methylphenyl] - piperazinyl] tetralin and (-) -trans-5-iodo-2-α-methoxy-α-trifluoromethylphenylacetoxy-3-[4-[2-methylphenyl]-piperazinyl]tetralin

155 mg (0.346 mmol) of (±)-trans-5-iodo-2-hydroxy-3-[4-[2-methylphenyl]piperazinyl]tetralin was dissolved in 3.6 ml of dichloromethane, and 1 mg (0.008 mmol) of 4-dimethylamino-pyridine (DMAP) and 48 μl (0.346 mmol) of triethylamine were added. The mixture was cooled to -80°C with stirring. 100 mg (0.369 mmol) of (-)-α-methoxy-α-trifluoro-methylphenylacetylchloride ((-)-MTPA-C1) was slowly added to the reaction solution. The reaction mixture was stirred for 30 minutes at -80°C and 24 hours at room temperature. The reaction was terminated by adding water and the reaction solution was extracted with a mixture of water/dichloromethane. The organic layer was dried over anhydrous sodium sulfate and the solvent was evaporated. The residue was purified by flash chromatography (dichloromethane) and the solvent was evaporated to obtain 62 mg (53.9%) of (+)-trans-5-iodo-2-α-methoxy-α-trifluoromethylphenyl-acetoxy-3-[4-[2-methylphenyl]piperazinyl]tetralin and 51 mg (44.4%) of (-) -trans-5-iodo-2-α-methoxy-α-trifluoromethylphenylacetoxy-3-[4-[2-methylphenyl]-piperazinyl]tetralin.

- (+)-trans-5-iodo-2-α-methoxy-α-trifluoromethyl-phenylacetoxy-3-[4-[2-methylphenyl]piperazinyl]tetralin
  TLC (dichloromethane:n-hexane = 1:1); Rf=0.48
  $[\alpha]^{23}_D$ = +23.8 (C = 1.0, chloroform)
- (-)-trans-5-iodo-2-α-methoxy-α-trifluoromethyl-phenylacetoxy-3-[4-[2-methylphenyl]piperazinyl]tetralin
  TLC (dichloromethane:n-hexane = 1:1); Rf = 0.37
  $[\alpha]^{23}_D$= -60.3 (C = 1.0, chloroform)

(2) Synthesis of (+)-trans-5-iodo-2-hydroxy-3-[4-[2-methylphenyl]piperazinyl]tetralin (DRE341)

62 mg (0.093 mmol) of (+)-trans-5-iodo-2-α-methoxy-α-trifluoromethylphenylacetoxy-3-[4-[2-methylphenyl] piperazin yl]tetralin was dissolved in 1 ml of tetrahydrofuran. After the addition of 5 ml of 6N sodium hydroxide, the mixture was stirred for 72 hours at room temperature. The reaction solution was extracted with a mixture of water/ dichloromethane, the organic layer was dried over anhydrous sodium sulfate, and the solvent was evaporated. The residue was purified by chromatography (dichloromethane → dichloromethane:methanol = 95:5) and the solvent was evaporated to obtain 4 mg (9.5%) of the title compound as a reddish brown amorphous substance.
$[\alpha]^{23}_D$= +53.7 (C = 1.0, chloroform)

(3) Synthesis of (-)-trans-5-iodo-2-hydroxy-3-[4-[2-methylphenyl]piperazinyl]tetralin (DRE342)

51 mg (0.077 mmol) of (-)-trans-5-iodo-2-α-methoxy-α-trifluoromethylphenylacetoxy-3-[4-[2-methylphenyl] piperazin yl]tetralin was dissolved in 1 ml of tetrahydrofuran. After the addition of 5 ml of 6N sodium hydroxide, the mixture was stirred for 72 hours at room temperature. The reaction solution was extracted with a mixture of water/ dichloromethane, the organic layer was dried over anhydrous sodium sulfate, and the solvent was evaporated. The residue was purified by chromatography (dichloromethane → dichloromethane:methanol = 95:5) and the solvent was evaporated to obtain 16 mg (44.4%) of the title compound as a reddish brown amorphous substance.
$[\alpha]^{23}_D$ = -44.5 (C = 1.0, chloroform)

Example 7

Synthesis of (-)-trans-5-tributylstannyl-2-hydroxy-3-[4-[2-methylphenyl]piperazinyl]tetralin (DRE377)

132 mg (0.294 mmol) of (-)-trans-5-iodo-2-hydroxy-3-[4-[2-methylphenyl]piperazinyl]tetralin was dissolved in 8 ml of toluene and argon gas was bubbled through the mixture for 5 minutes to deaerate the mixture. After the addition

of 318 μl (0.629 mmol) of bis(tributyltin) and 20 mg (0.017 mmol) of tetrakistriphenylphosphine palladium, the reaction solution was refluxed for 19 hours in an argon gas atmosphere. After removing the solvent by evaporation, the residue was purified by chromatography (dichloro methane → dichloromethane:ethyl acetate = 7:3) and NH chromatography (dichloromethane:ethyl acetate = 7:3). The solvent was evaporated to obtain 61 mg (33.9%) of the title compound as an orange amorphous substance. $^1$H-NMR($CDCl_3$)δ:

6.90-7.26(m,7H), 3.70-3.92(m,1H), 2.53-3.37(m,13H),
2.25(s,3H), 0.74-1.52(m,27H)

TLC (dichloromethane: ethyl acetate = 7:3); Rf = 0.68
$[\alpha]^{23}_D$ = -30.8 (C = 1.0, chloroform)

Example 8

(+)-trans-5-iodo-2-hydroxy-3-(4-phenyl-1-piperazinyl)-tetralin and (-)-trans-5-iodo-2-hydroxy-3-(4-phenyl-1-piperazinyl)tetralin (DRC140)

(1) Synthesis of (±)-trans-5-amino-2-hydroxy-3-(4-phenyl-1-piperazinyl)tetralin

950 mg (3.69 mmol) of N-(trifluoroacetyl)-1-amino-5,8-dihydronaphthaleneoxide and 1.55 g (9.55 mmol) of 1-phenylpiperazine were dissolved in 12.5 ml of ethanol and the mixture was refluxed for 20 hours. After leaving the mixture to cool for 24 hours, (+)-trans-5-amino-3-hydroxy-2-(4-phenyl-1-piperazinyl)tetralin with high polarity (a considerable quantity of (±)-trans-5-amino-2-hydroxy-3-(4-phenyl-1-piperazinyl)tetralin was mixed) was crystallized and separated by filtration. After removing the solvent by evaporation, the residue was purified by column chromatography (dichloromethane:ethyl acetate = 9:1) to obtain (±)-trans-5-amino-2-hydroxy-3-(4-phenyl-1-piperazinyl)tetr alin. The crystals previously separated by filtration were dissolved in a small amount of methylene chloride and purified using column chromatography (dichloromethane:ethyl acetate = 9:1). Low polar (±)-trans-5-amino-2-hydroxy-3-(4-phenyl-1-piperazinyl)tetralin was first removed, and high polar (±)-trans-5-amino-3-hydroxy-2-(4-phenyl-1-piperazinyl)tetralin was purified. The solvents were evaporated from these derivatives to obtain 411 mg (34.4%) of white crystals of (±)-trans-5-amino-2-hydroxy-3-(4-phenyl-1-piperazinyl) tetralin and 486 mg (40.7%) of skin color crystals of (±)-trans-5-amino-3-hydroxy-2-(4-phenyl-1-piperazinyl)-tetralin.

(2) Synthesis of (±)-trans-5-iodo-2-hydroxy-3-(4-phenyl-1-piperazinyl)tetralin

120 mg (0.371 mmol) of (±)-trans-5-amino-2-hydroxy-3-(4-phenyl-1-piperazinyl)tetralin was dissolved in a mixed solution of 2 ml of acetic acid and 1 ml of concentrated hydrochloric acid, and cooled to 5°C or less. A solution of 27 mg of sodium nitrite (0.395 mmol in 2 ml of water) was added to the cold solution while maintaining the temperature at 10°C or less. The mixture was stirred for 20 minutes at 10°C or less. A solution of potassium iodide (74 mg(0.447 mmol)) and iodine (57 mg (0.223 mmol)) in 1 ml of water was slowly added dropwise while controlling the temperature at 10°C or less. The solution was stirred for 3 hours at at 10°C or less, and then overnight at room temperature. The reaction solution was neutralized with sodium hydrogencarbonate and extracted with methylene chloride (50 ml × 5 times). The organic layer was washed with 10% sodium hydrogensulfite and dried over anhydrous sodium sulfate. The solvent was evaporated and the residue was purified by column chromatography (dichloromethane: ethyl acetate = 95:5). The solvent was evaporated to obtain 118 mg (73.3%) of yellow crystals.
mp; 165.3°C
$^1$H-NMR($CDCl_3$)δ;

2.90-2.62(5H,m), 3.09-3.00(2H,m), 3.30-3.14(5H,m),
3.48(1H,q,J=20.5Hz), 3.19-3.84(1H,m), 4.16(1H,s),
6.94-6.83(4H,m), 6.97(1H,d,J=8.30Hz), 7.11(1H,d,J=7.81Hz),
7.31-7.14(2H,m), 7.71(1H,d,J=7.81Hz)

FAB/MS(M/Z); 435($M+H^+$)

(3) Synthesis of (-),(+)-trans-5-iodo-2-α-methoxy-α-trifluoromethylphenylacetoxy-3-(4-phenyl-1-piperazinyl)-tetralin

300 mg (0.691 mmol) of (±)-trans-5-iodo-2-hydroxy-3-(4-phenyl-1-piperazinyl)tetralin was dissolved in 5.0 ml of dichloromethane and 2.0 mg (0.016 mmol) of 4-dimethylamino-pyridine (DMAP) and 70 mg (0.692 mmol) of triethylamine were added. The mixture was stirred while cooling to -80°C. 200 mg (0.792 mmol) of (-)-α-methoxy-α-trifluoromethylphenylacetylchloride was slowly added to the reaction solution. The mixture was allowed to cool to room temperature and stirred for 24 hours. The reaction was terminated by the addition of 10 ml of water and the reaction solution was extracted with dichloromethane.

The organic layer was washed with saturated brine and dried over anhydrous sodium sulfate. After evaporating the solvent, the residue was purified by flash column chromatography (dichloromethane). Low polar (+)-trans-5-iodo-2-α-methoxy-α-trifluoromethylphenylacetoxy-3-(4-phenyl-1-piperazinyl)tetralin was first removed, and high polar (-)-trans-5-iodo-2-α-methoxy-α-trifluoromethylphenylacetoxy-3-(4-phenyl-1-piperazinyl)-tetralin removed next. The solvents were evaporated from these two optical isomers to obtain 154 mg (68.5%) of a light yellow oily substance of (-)-trans-5-iodo-2-α-methoxy-α-trifluoromethylphenylacetoxy-3-(4-phenyl-1-piperazinyl)-tetralin and 194 mg (86.3%) of a light yellow oily substance of (+)-trans-5-iodo-2-α-methoxy-α-trifluoromethylphenyl-acetoxy-3-(4-phenyl-1-piperazinyl) tetralin.

- (-)-trans-5-iodo-2-α-methoxy-α-trifluoromethylphenylacetoxy-3-(4-phenyl-1-piperazinyl)-tetralin
  $^1$H-NMR($CDCl_3$)δ;
  2.66-2.73(2H,m), 2.76-3.28(11H,m), 3.56(3H,s),
  5.46-5.52(1H,m), 6.84-6.91(4H,m), 7.03-7.15(1H,m),
  7.24-7.29(3H,m), 7.34-7.39(3H,m), 7.58-7.60(1H,m),
  7.67-7.73(1H,m)
  $[\alpha]^{23}_D$= -44.2 (c = 1.0, chloroform)
  TLC(dichloromethane); Rf = 0.36
- (+)-trans-5-iodo-2-α-methoxy-α-trifluoromethylphenylacetoxy-3-(4-phenyl-1-piperazinyl)-tetralin
  $^1$H-NMR($CDCl_3$)δ;
  2.73-2.78(2H,m), 2.82-3.23(11H,m), 3.63(3H,s),
  5.47-5.57(1H,m), 7.02(1H,d)J=7.82Hz, 7.22-7.29(2H,m),
  7.35-7.47(3H,m), 7.66-7.72(3H,m)
  $[\alpha]^{23}_D$= +30.7 (c = 1.0, chloroform)
  TLC (dichloromethane); Rf = 0.49

(4) Synthesis of (-)-trans-5-iodo-2-hydroxy-3-(4-phenyl-1-piperazinyl)tetralin (DRC140)

154 mg (0.237 mmol) of (-)-trans-5-iodo-2-α-methoxy-α-trifluoromethylphenylacetoxy-3-(4-phenyl-1-piperazinyl)-tetralin was dissolved in 5 ml of tetrahydrofuran. After the addition of 8 ml of 4N sodium hydroxide, the mixture was stirred for 24 hours at room temperature. After removing tetrahydrofuran by evaporation, the residue was extracted with a water/dichloromethane mixture. The organic layer was washed with saturated brine and dried over anhydrous sodium sulfate. After evaporating the solvent, the residue was purified by column chromatography (dichloromethane → dichloromethane:methanol = 8:2). The solvent was removed by evaporation to obtain 66 mg (64.1%) of light yellow crystals of (-)-trans-5-iodo-2-hydroxy-3-(4-phenyl-1-piperazinyl)-tetralin.
$^1$H-NMR($CDCl_3$) δ;
2.62-2.90(5H,m), 3.00-3.09(2H,m), 3.14-3.30(5H,m),
3.48(1H,q,J=20.5Hz), 3.19-3.84(1H,m), 4.16(1H,s),
6.83-6.94(4H,m), 6.97(1H,d,J=8.30Hz),
7.11(1H,d,J=7.81Hz), 7.14-7.31(2H,m), 7.71(1H,d)J=7.81Hz
$[\alpha]^{23}_D$ = -64.6 (C = 1.0, chloroform)

(5) Synthesis of (+)-trans-5-iodo-2-hydroxy-3-(4-phenyl-1-piperazinyl)tetralin

194 mg (0.298 mmol) of (+)-trans-5-iodo-2-α-methoxy-α-trifluoromethylphenylacetoxy-3-(4-phenyl-1-piperazinyl)-tetralin was dissolved in 5 ml of tetrahydrofuran. After the addition of 8 ml of 4N sodium hydroxide, the mixture was stirred for 24 hours at room temperature. After removing tetrahydrofuran by evaporation, the residue was extracted with a water/dichloromethane mixture. The organic layer was washed with saturated brine and dried over anhydrous sodium sulfate. After evaporating the solvent, the residue was purified by column chromatography (dichloromethane → dichloromethane:methanol = 8:2). The solvent was removed by evaporation to obtain 88 mg (68.0%) of light yellow crystals of (+)-trans-5-iodo-2-hydroxy-3-(4-phenyl-1-piperazinyl)-tetralin.
$^1$H-NMR($CDCl_3$) δ;
2.62-2.90(5H,m), 3.00-3.09(2H,m), 3.14-3.30(5H,m),
3.48(1H,q)J=20.5Hz, 3.19-3.84(1H,m), 4.16(1H,s),
6.83-6.94(4H,m), 6.97(1H,d,J=8.30Hz),
7.11(1H,d,J=7.81Hz), 7.14-7.31(2H,m),
7.71(1H,d,J=7.81Hz)
$[\alpha]^{23}_D$ = +74.6 (C = 1.0, chloroform)

Example 9

(±)-trans-5-iodo-2-hydroxy-3-[4-[5-methoxy-2-methylphenyl] piperazinyl]tetralin (DRE378)

(1) Synthesis of 1-[5-methoxy-2-methylphenyl]piperazine

15 ml of n-buthanol and 1.0 g (7.290 mmol) of 5-methoxy-2-ethylaniline were added to 1.3 g (7.283 mmol) of bis(2-chloroethyl)amine hydrochloride. The mixture was heated with stirring to dissolve bis(2-chloroethyl)- amine hydrochloride. 1.6 g (15.096 mmol) of sodium carbonate was added to the reaction solution and the mixture was refluxed for 24 hours. After cooling, 2N sodium hydroxide was added and the mixture was extracted with a water/dichloromethane mixture. The organic layer was dried over anhydrous sodium sulfate and the solvent was evaporated under vacuum. The residue was purified by chromatography (dichloromethane:methanol = 95:5 → dichloromethane:methanol = 8:2) and the solvent was evaporated to obtain 885 mg (59.0%) of the title compound as brown crystals. TLC (dichloromethane:methanol = 95:5); Rf = 0.18

(2) Synthesis of (±)-trans-5-amino-2-hydroxy-3-[4-[5-methoxy-2-methylphenyl]piperazinyl]tetralin

20 ml of ethanol was added to 885 mg (4.290 mmol) of 1-(5-methoxy-2-methylphenyl)piperazine to dissolve the latter. 1.1 g (4.277 mmol) of N-[trifluoroacetyl]-1-amino-5,8-dihydronaphthalene oxide was added to the reaction solution and the mixture was refluxed for 16 hours. After cooling the reaction solution, the solvent was removed by evaporation and the residue was dissolved in 5 ml of methanol. 6 ml of 2N sodium hydroxide was added to the solution and the mixture was stirred for 20 hours. The reaction solution was extracted with a mixture of water and dichloromethane, the organic layer was dried over anhydrous sodium sulfate, and the solvent was evaporated. The residue was purified by flash chromatography (dichloromethane:methanol = 98:2) to obtain 626 mg (39.9%) of (±)-trans-5-amino-2-hydroxy-3-[4-[5-methoxy-2-methylphenyl]piperazinyl]tetralin as a light yellow amorphous low polar substance.
TLC (dichloromethane:methanol = 97:3); Rf = 0.68
FAB/MS(m/z); 368($MH^+$)

(3) Synthesis of (±)-trans-5-iodo-2-hydroxy-3-[4-[5-methoxy-2-methylphenyl]piperazinyl]tetralin (DRE378)

3 ml of acetic acid and 1.5 ml of concentrated hydrochloric acid were added to 200 mg (0.544 mmol) of (±)-trans-5-amino-2-hydroxy-3-[4-[5-methoxy-2-methylphenyl]piperazinyl]tetralin and the mixture was stirred to cool with ice to a temperature of 5°C or less. A solution of 38 mg of sodium nitrite (0.551 mmol in 3 ml of water) was slowly added dropwise while maintaining the temperature of the reaction solution at 7°C or less. The reaction solution was stirred for 20 minutes while controlling the temperature at 5°C. Next, a solution of potassium iodide (112 mg (0.675 mmol)) and iodine (85 mg (0.335 mmol)) in 1.5 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane: ethyl acetate = 9:1) to obtain 103 mg (39.6%) of the title compound as light purple crystals.
$^1$H-NMR($CDCl_3$) δ:
7.71(d, J=7.82Hz,1H), 6.48-7.25(m,5H), 3.69-3.99(m,4H),
2.64-3.51(m,13H), 2.26(s,3H)
TLC (dichloromethane:methanol = 98:2); Rf = 0.63
FAB/MS(m/z); 479($MH^+$)

Example 10

(±)-trans-5-iodo-2-hydroxy-3-[4-[5-hydroxy-2-methylphenyl] piperazinyl]tetralin

(1) Synthesis of (±)-trans-5-amino-2-hydroxy-3-[4-[5-hydroxy-2-methylphenyl]piperazinyl]tetralin

10 ml of 48% HBr was added to 425 mg (1.156 mmol) of (±)-trans-5-amino-2-hydroxy-3-[4-[5-methoxy-2-methylphenyl ]piperazinyl]tetralin and the mixture was refluxed for 90 hours. The reaction solution was alkalinized with 6N sodium hydroxide while stirring and cooling with ice. The reaction solution was extracted with a mixture of water and dichloromethane, the organic layer was dried over anhydrous sodium sulfate, and the solvent was evaporated. The residue was purified by flash chromatography (dichloromethane:methanol = 98:2) and the solvent was evaporated to obtain 242 mg (59.2%) of the title compound as a brown amorphous substance.
TLC (dichloromethane:methanol = 97:3); Rf = 0.30

(2) Synthesis of (±)-trans-5-iodo-2-hydroxy-3-[4-[5-hydroxy-2-methylphenyl]piperazinyl]tetralin

4 ml of acetic acid and 2 ml of concentrated hydrochloric acid were added to 242 mg (0.685 mmol) of (±)-trans-5-amino-2-hydroxy-3-[4-[5-hydroxy-2-methylphenyl]piperazinyl]tetralin and the mixture was stirred to cool with ice to a temperature of 5°C or less. A solution of 54 mg of sodium nitrite (0.783 mmol in 4 ml of water) was slowly added dropwise while maintaining the temperature of the reaction solution at 7°C or less. The reaction solution was stirred for 20 minutes while controlling the temperature at 5°C. Next, a solution of potassium iodide (140 mg (0.843 mmol)) and iodine (104 mg (0.410 mmol)) in 2 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 97:3) to obtain 142 mg (44.7%) of the title compound as a reddish brown amorphous substance.
$^1$H-NMR($CDCl_3$)δ:
7.83(d,J=7.57Hz,1H), 6.54-7.43(m,5H), 3.74-3.98(m,1H),
2.15-3.39(m,13H), 2.22(s,3H)
TLC (dichloromethane:methanol = 97:3); Rf = 0.44
FAB/MS(m/z); 465($MH^+$)

Example 11

(±)-trans-5-iodo-2-hydroxy-3-[4-[2-pyridyl]piperazinyl]-tetralin (DRE328)

(1) Synthesis of (±)-trans-5-amino-2-hydroxy-3-[4-[2-pyridyl]piperazinyl]tetralin and (±)-trans-8-amino-2-hydroxy-3-[4-[2-pyridyl]-piperazinyl]tetralin

25 ml of ethanol was added to 1.14 ml (7.823 mmol) of 1-(2-pyridyl)piperazine to dissolve the piperazine compound. 1.0 g (3.888 mmol) of N-[trifluoroacetyl]-1-amino-5,8-dihydronaphthalene oxide was added to the reaction solution and the mixture was refluxed for 21 hours. After cooling the reaction solution, the solvent was removed by evaporation and the residue was dissolved in 10 ml of methanol. 20 ml of 2N sodium hydroxide was added to the solution and the mixture was stirred for 20 hours. The reaction solution was extracted with a mixture of water/dichloromethane, the organic layer was dried over anhydrous sodium sulfate, and the solvent was evaporated. The residue was purified by flash chromatography (dichloromethane:ethyl acetate = 7:3) to obtain 570 mg (40.4%) of (±)-trans-5-amino-2-hydroxy-3-[4-[2-pyridyl]-piperazinyl]tetralin as a light yellow amorphous low polar substance and 432 mg (34.3%) of (+)-trans-8-amino-2-hydroxy-3-[4-[2- pyridyl]piperazinyl]tetralin as a light yellow amorphous high polar substance.

- (±)-trans-5-amino-2-hydroxy-3-[4-[2-pyridyl]piperazinyl]-tetralin
  TLC (dichloromethane:methanol = 98:2); Rf = 0.49
  FAB/MS(m/z);325($MH^+$)

- (±)-trans-8-amino-2-hydroxy-3-[4-[2-pyridyl]piperazinyl]-tetralin
  TLC (dichloromethane:methanol = 98:2); Rf = 0.28
  FAB/MS (m/z) ; 325 ($MH^+$)

(2) Synthesis of (±)-trans-5-iodo-2-hydroxy-3-[4-[2-pyridyl]-piperazinyl]tetralin (DRE328)

4 ml of acetic acid and 2 ml of concentrated hydrochloric acid were added to 240 mg (0.744 mmol) of (±)-trans-5-amino-2-hydroxy-3-[4-[2-pyridyl]piperazinyl]tetralin and the mixture was stirred to cool with ice to a temperature of 5°C or less. A solution of 54 mg of sodium nitrite (0.783 mmol in 4 ml of water) was slowly added dropwise while maintaining the temperature of the reaction solution at 7°C or less. The reaction solution was stirred for 20 minutes while controlling the temperature at 5°C. Next, a solution of potassium iodide (148 mg (0.892 mmol)) and iodine (114 mg (0.449 mmol)) in 2 ml of water was slowly added dropwise while controlling the temperature at 7°C or less. The

reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 97:3) to obtain 202 mg (62.4%) of the title compound as a brown amorphous substance.
$^1$H-NMR($CDCl_3$)δ:

8.18(d,J=5.12Hz,1H), 7.39-7.72(m,2H), 6.81-7.26(m,2H), 6.56-6.70(m,2H), 3.76-4.06(m,1H), 3.13-3.70(m,6H), 2.39-3.08(m,7H)

TLC (dichloromethane:methanol = 97:3); Rf = 0.65
FAB/MS(m/z);436($MH^+$)

Example 12

(±)-trans-5-iodo-2-hydroxy-3-[4-[3-methylphenyl]-piperazinyl]tetralin (DRF380)

(1) Synthesis of (±)-trans-5-amino-2-hydroxy-3-[4-[3-methylphenyl]piperazinyl]tetralin and (±)-trans-8-amino-2-hydroxy-3-[4-[3-methylphenyl]-piperazinyl]tetralin

20 ml of ethanol and 2.16 ml of triethylamine were added to 1.94 g (7.785 mmol) of 1-(3-tolyl)piperazine hydrochloride and dissolved by heating while stirring. 1.0 g (3.888 mmol) of N-[trifluoroacetyl]-1-amino-5,8-dihydronaphthalene oxide was added to the reaction solution and the mixture was refluxed for 20 hours. After cooling the reaction solution, the solvent was removed by evaporation and the residue was dissolved in 7 ml of methanol. 20 ml of 2N sodium hydroxide was added to the solution and the mixture was stirred for 20 hours. The reaction solution was extracted with a mixture of water/dichloromethane, the organic layer was dried over anhydrous sodium sulfate, and the solvent was evaporated. The residue was purified by flash chromatography (dichloromethane:ethyl acetate = 7:3) to obtain 538 mg (41.1%) of (±)-trans-5-amino-2-hydroxy-3-[4-[3-methylphenyl]piperazinyl]tetralin as a white amorphous low polar substance and 379 mg (28.9%) of (+)-trans-8-amino-2-hydroxy-3-[4-[3-methylphenyl]piperazinyl]tetralin as a light brown amorphous high polar substance.

- (±)-trans-5-amino-2-hydroxy-3-[4-[3-methylphenyl]-piperazinyl]tetralin
  TLC (dichloromethane: ethyl acetate = 7:3); Rf = 0.50
- (±)-trans-8-amino-2-hydroxy-3-[4-[3-methylphenyl]-piperazinyl]tetralin
  TLC (dichloromethane: ethyl acetate = 7:3); Rf = 0.32

(2) Synthesis of (±)-trans-5-iodo-2-hydroxy-3-[4-[3-methylphenyl]piperazinyl]tetralin (DRF380)

8 ml of acetic acid and 4 ml of concentrated hydrochloric acid were added to 538 mg (1.594 mmol) of (±)-trans-5-amino-2-hydroxy-3-[4-[3-methylphenyl]piperazinyl]tetralin and the mixture was stirred to cool with ice to a temperature of 5°C or less. A solution of 120 mg of sodium nitrite (1.739 mmol in 8 ml of water) was slowly added dropwise while maintaining the temperature of the reaction solution at 7°C or less. The reaction solution was stirred for 20 minutes while controlling the temperature at 5°C. Next, a solution of potassium iodide (320mg (1.928 mmol)) and iodine (244 mg (0.961 mmol)) in 4 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 98:1) to obtain 531 mg (77.0%) of the title compound as dark green crystals.
$^1$H-NMR($CDCl_3$)δ:

7.69(d,J=7.08Hz,1H), 6.67-7.23(m,6H), 3.71-4.00(m,1H), 2.61-3.46(m,13H), 2.32(s,3H)

TLC (dichloromethane:methanol = 97:3); Rf = 0.58
FAB/MS(m/z);449($MH^+$)

Example 13

(±)-trans-5-iodo-2-hydroxy-3-[4-[4-methylphenyl]-piperazinyl]tetralin (DRF381)

(1) Synthesis of (±)-trans-5-amino-2-hydroxy-3-[4-[4-methylphenyl]piperazinyl]tetralin and (±)-trans-8-amino-2-hydroxy-3-[4-[4-methylphenyl]-piperazinyl]tetralin

20 ml of ethanol and 2.16 ml of triethylamine were added to 1.94 g (7.785 mmol) of 1-(4-tolyl)piperazine hydrochloride and dissolved by heating while stirring. 1.0 g (3.888 mmol) of N-[trifluoroacetyl]-1-amino-5,8-dihydronaphthalene oxide was added to the reaction solution and the mixture was refluxed for 20 hours. After cooling the reaction solution, the solvent was removed by evaporation and the residue was dissolved in 7 ml of methanol. 20 ml of 2N sodium hydroxide was added to the solution and the mixture was stirred for 20 hours. The reaction solution was extracted with a mixture of water and dichloromethane, the organic layer was dried over anhydrous sodium sulfate, and the solvent was evaporated. The residue was purified by chromatography (dichloromethane:ethyl acetate = 7:3) to obtain 574 mg (43.8%) of (±)-trans-5-amino-2-hydroxy-3-[4-[4-methylphenyl]piperazinyl]tetralin as a white amorphous low polar substance and 450 mg (34.4%) of (±)-trans-8-amino-2-hydroxy-3-[4-[4-methylphenyl] piperazinyl]tetralin as a light brown amorphous high polar substance.

- (±)-trans-5-amino-2-hydroxy-3-[4-[4-methylphenyl]-piperazinyl]tetralin
  TLC (dichloromethane:methanol = 98:2); Rf = 0.40
- (±)-trans-8-amino-2-hydroxy-3-[4-[4-methylphenyl]-piperazinyl]tetralin
  TLC (dichloromethane:methanol = 98:2); Rf = 0.28

(2) Synthesis of (±)-trans-5-iodo-2-hydroxy-3-[4-[4-methylphenyl]piperazinyl]tetralin (DRF381)

7 ml of acetic acid and 3.5 ml of concentrated hydrochloric acid were added to 547 mg (1.701 mmol) of (±)-trans-5-amino-2-hydroxy-3-[4-[4-methylphenyl]-piperazinyl]tetralin and the mixture was stirred to cool with ice to a temperature of 5°C or less. 127 mg of sodium nitrite (1.841 mmol in 7 ml of water) was slowly added dropwise while maintaining the temperature of the reaction solution at 7°C or less. The reaction solution was stirred for 20 minutes while controlling the temperature at 5°C. Next, a solution of potassium iodide (340 mg (2.048mmol)) and iodine (260 mg (1.024 mmol)) in 3.5 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 99:1) to obtain 589 mg (77.2%) of the title compound as dark brown crystals.
$^1$H-NMR($CDCl_3$) δ:
7.69(d,J=7.57Hz,1H), 6.72-7.23(m,6H), 3.70-3.98(m,1H),
2.51-3.44(m,13H), 2.27(s,3H)
TLC (dichloromethane:methanol = 97:3); Rf = 0.67
FAB/MS(m/z);449($MH^+$)

Example 14

(+)-trans-5-iodo-2-hydroxy-3-[4-[2-ethylphenyl]-piperazinyl]tetralin (DRE353)

(1) Synthesis of 1-[2-ethylphenyl]piperazine

10 ml of n-buthanol and 629 μl (5.607 mmol) of 2-ethylaniline were added to 1.0 g (5.603 mmol) of bis(2-chloroethyl)amine hydrochloride. The mixture was heated with stirring to dissolve bis(2-chloroethyl)amine hydrochloride. 1.2 g (11.322 mmol) of sodium carbonate was added to the reaction solution and the mixture was refluxed for 24 hours. After cooling, 2N sodium hydroxide was added and the mixture was extracted with a water/dichloromethane mixture. The organic layer was dried over anhydrous sodium sulfate and the solvent was evaporated. The residue was purified by chromatography (dichloromethane:methanol = 97:3 → dichloromethane:methanol = 9:1) and the solvent was evaporated to obtain 519 mg (48.5%) of the title compound as a brown oily substance.
TLC (dichloromethane:methanol = 97:3); Rf = 0.10
FAB/MS (m/z) ; 191 ($MH^+$)

(2) Synthesis of (+)-trans-5-amino-2-hydroxy-3-[4-[2-ethylphenyl]piperazinyl]tetralin and (±)-trans-8-amino-2-hydroxy-3-[4-[2-ethylphenyl]-piperazinyl]tetralin

20 ml of ethanol was added to 941 mg (4.945 mmol) of 1-(2-ethylphenyl)piperazine to dissolve the piperazine. 1.0 g (3.888 mmol) of N-[trifluoroacetyl]-1-amino-5,8-dihydronaphthalene oxide was added to the reaction solution and the mixture was refluxed for 21 hours. After cooling the reaction solution, the solvent was removed by evaporation and the residue was dissolved in 7 ml of methanol. 20 ml of 2N sodium hydroxide was added to the solution and the mixture was stirred for 20 hours. The reaction solution was extracted with a mixture of water and dichloromethane, the organic layer was dried over anhydrous sodium sulfate, and the solvent was evaporated. The residue was purified by flash chromatography (dichloromethane:ethyl acetate = 7:3) to obtain 508 mg (37.2%) of (+)-trans-5-amino-2-hydroxy-3-[4-[2-ethylphenyl-piperazinyl]tetralin as a light yellow amorphous low polar substance and 530 mg (38.8%) of (±)-trans-8-amino-2-hydroxy-3-[4-[2- ethylphenyl]piperazinyl]tetralin as a light yellow amorphous high polar substance.

- (±)-trans-5-amino-2-hydroxy-3-[4-[2-ethylphenyl]-piperazinyl]tetralin
  TLC (dichloromethane:methanol = 98:2); Rf = 0.49
- (±)-trans-8-amino-2-hydroxy-3-[4-[2-ethylphenyl]-piperazinyl]tetralin
  TLC (dichloromethane:methanol = 98:2); Rf = 0.28

(3) Synthesis of (±)-trans-5-iodo-2-hydroxy-3-[4-[2-ethylphenyl]piperazinyl]tetralin (DRE353)

8 ml of acetic acid and 4 ml of concentrated hydrochloric acid were added to 500 mg (1.423 mmol) of (±)-trans-5-amino-2-hydroxy-3-[4-[2-ethylphenyl]piperazinyl]tetralin and the mixture was stirred to cool with ice to a temperature of 5°C or less. A solution of 110 mg of sodium nitrite (1.594 mmol in 8 ml of water) was slowly added dropwise while maintaining the temperature of the reaction solution at 7°C or less. The reaction solution was stirred for 20 minutes while controlling the temperature at 5°C. Next, a solution of potassium iodide (292 mg(1.758 mmol)) and iodine (222 mg (0.874 mmol)) in 4 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 98:2) to obtain 483 mg (73.4%) of the title compound as a yellowish brown crystals.
$^1$H-NMR($CDCl_3$)δ:
7.70(d,J=7.81Hz,1H), 6.82-7.27(m,5H), 3.77-4.09(m,1H),
2.63-3.44(m,16H), 1.27(t,J=14.89Hz,3H)
TLC (dichloromethane:methanol = 97:3); Rf = 0.65
FAB/MS(m/z); 463($MH^+$)

Example 15

(±)-trans-5-iodo-2-hydroxy-3-[4-[2,3-dimethylphenyl]-piperazinyl]tetralin (DRE354)

(1) Synthesis of 1-[2,3-dimethylphenyl]piperazine

20 ml of n-buthanol and 1.37 ml (11.226 mmol) of 2,3-dimethylaniline were added to 2.0 g (11.205 mmol) of bis(2-chloroethyl)amine hydrochloride. The mixture was heated with stirring to dissolve bis(2-chloroethyl)-amine hydrochloride. 2.3 g (21.700 mmol) of sodium carbonate was added to the reaction solution and the mixture was refluxed for 24 hours. After cooling, 2N sodium hydroxide was added and the mixture was extracted with a water/dichloromethane mixture. The organic layer was dried over anhydrous sodium sulfate and the solvent was evaporated. The residue was purified by chromatography (dichloromethane:methanol = 95:5 → dichloromethane:methanol = 8:2) and the solvent was evaporated to obtain 1.16 g (54.3%) of the title compound as a brown oily substance.
TLC (dichloromethane:methanol = 95:5); Rf = 0.10
FAB/MS (m/z) ; 191 ($MH^+$)

(2) Synthesis of (±)-trans-5-amino-2-hydroxy-3-[4-[2,3-dimethylphenyl]piperazinyl]tetralin and (±)-trans-8-amino-2-hydroxy-3-[4-[2,3-dimethylphenyl]-piperazinyl]tetralin

40 ml of ethanol was added to 1.5 g (7.883 mmol) of 1-(2,3-dimethylphenyl)piperazine to dissolve the piperazine. 2.0 g (7.776 mmol) of N-[trifluoroacetyl]-1-amino-5,8-dihydronaphthalene oxide was added to the reaction solution and the mixture was refluxed for 22 hours. After cooling the reaction solution, the solvent was removed by evaporation and the residue was dissolved in 14 ml of methanol. 30 ml of 2N sodium hydroxide was added to the solution and the mixture was stirred for 20 hours. The reaction solution was extracted with a mixture of water and dichloromethane, the organic layer was dried over anhydrous sodium sulfate, and the solvent was evaporated. The residue was purified by flash chromatography (dichloromethane:methanol = 98:2) to obtain 681 mg (24.6%) of (±)-trans-5-amino-2-hydroxy-3-[4-[2,3-dimethylphenyl]-piperazinyl]tetralin as a light yellow amorphous low polar substance and 569 mg (20.5%) of (+)-trans-8-amino-2-hydroxy-3-[4-[2,3-dimethylphenyl]piperazinyl]tetralin as a light yellow amorphous high polar substance.

- (±)-trans-5-amino-2-hydroxy-3-[4-[2,3-dimethylphenyl]-piperazinyl]tetralin
  TLC (dichloromethane:methanol = 97:3); Rf = 0.60
- (±)-trans-8-amino-2-hydroxy-3-[4-[2,3-dimethylphenyl]-piperazinyl]tetralin
  TLC (dichloromethane:methanol = 97:3); Rf = 0.40

(3) Synthesis of (±)-trans-5-iodo-2-hydroxy-3-[4-[2,3-dimethylphenyl]piperazinyl]tetralin (DRE354)

10 ml of acetic acid and 5 ml of concentrated hydrochloric acid were added to 668 mg (1.900 mmol) of (±)-trans-5-amino-2-hydroxy-3-[4-[2,3-dimethylphenyl]piperazinyl]tetralin and the mixture was stirred to cool with ice to a temperature of 5°C or less. A solution of 150 mg of sodium nitrite (2.174 mmol in 10 ml of water) was slowly added dropwise while maintaining the temperature of the reaction solution at 7°C or less. The reaction solution was stirred for 20 minutes while controlling the temperature at 5°C. Next, a solution of potassium iodide (390 mg (2.349 mmol)) and iodine (290 mg (1.143 mmol)) in 5 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane:methanol = 98:2) to obtain 668 mg (76.1%) of the title compound as a brown amorphous substance.
$^1$H-NMR($CDCl_3$)δ:
7.69(d,J=7.57Hz,1H), 6.81-7.21(m,5H), 3.69-4.00(m,1H),
2.64-3.37(m,13H), 2.26(s,6H)
TLC (dichloromethane:methanol = 98:2); Rf = 0.58
FAB/MS (m/z) ; 463 ($MH^+$)

Example 16

(±)-trans-5-iodo-2-hydroxy-3-[4-[2,6-dimethylphenyl]-piperazinyl]tetralin (DRF382)

(1) Synthesis of 1-[2,6-dimethylphenyl]piperazine

40 ml of n-buthanol and 2.8 ml (22.644 mmol) of 2,6-dimethylaniline were added to 4.0 g (22.410 mmol) of bis(2-chloroethyl)amine hydrochloride. The mixture was heated with stirring to dissolve bis(2-chloroethyl)-amine hydrochloride. 4.8 g (45.287 mmol) of sodium carbonate was added to the reaction solution and the mixture was refluxed for 24 hours. After cooling, 2N sodium hydroxide was added and the mixture was extracted with a water/dichloromethane mixture. The organic layer was dried over anhydrous sodium sulfate and the solvent was evaporated. The residue was purified by chromatography (dichloromethane:methanol = 95:5 → dichloromethane:methanol = 8:2) and the solvent was evaporated to obtain 2.13 g (50.0%) of the title compound as a brown oil.
TLC (dichloromethane:methanol = 95:5); Rf = 0.13
FAB/MS(m/z); 191($MH^+$)

(2) Synthesis of (±)-trans-5-amino-2-hydroxy-3-[4-[2,6-dimethylphenyl]piperazinyl]tetralin and (±)-trans-8-amino-2-hydroxy-3-[4-[2,6-dimethylphenyl]-piperazinyl]tetralin

30 ml of ethanol was added to 2.13 g (11.193 mmol) of 1-(2,6-dimethylphenyl)piperazine to dissolve the piperazine. 1.5 g (5.832 mmol) of N-[trifluoroacetyl]-1-amino-5,8-dihydronaphthalene oxide was added to the reaction solution and the mixture was refluxed for 18 hours. After cooling the reaction solution, the solvent was removed by evaporation and the residue was dissolved in 7 ml of methanol. 25 ml of 2N sodium hydroxide was added to the reaction solution and the mixture was stirred for 20 hours. The reaction solution was extracted with a water/dichloromethane

mixture, the organic layer was dried over anhydrous sodium sulfate, and the solvent was evaporated. The residue was purified by flash chromatography (dichloromethane:methanol = 98:2) to obtain 965 mg (47.1%) of (±)-trans-5-amino-2-hydroxy-3-[4-[2,6-dimethylphenyl]piper azinyl]tetralin as a light yellow amorphous low polar substance and 796 mg (38.8%) of (±)-trans-8-amino-2-hydroxy-3-[4-[2,6-dimethylphenyl]piperazinyl]tetralin as a light yellow amorphous high polar substance.

- (±)-trans-5-amino-2-hydroxy-3-[4-[2,6-dimethylphenyl]-piperazinyl]tetralin
  TLC (dichloromethane:methanol = 97:3); Rf = 0.68
- (±)-trans-8-amino-2-hydroxy-3-[4-[2,6-dimethylphenyl]-piperazinyl]tetralin
  TLC (dichloromethane:methanol = 97:3); Rf = 0.43

(3) Synthesis of (±)-trans-5-iodo-2-hydroxy-3-[4-[2,6-dimethylphenyl]piperazinyl]tetralin (DRF382)

14 ml of acetic acid and 7 ml of concentrated hydrochloric acid were added to 965 mg (2.745 mmol) of (±)-trans-5-amino-2-hydroxy-3-[4-[2,6-dimethylphenyl]piperazinyl]tetralin and the mixture was stirred to cool with ice to a temperature of 5°C or less. A solution of 217 mg of sodium nitrite (3.145 mmol in 14 ml of water) was slowly added dropwise while maintaining the temperature of the reaction solution at 7°C or less. The reaction solution was stirred for 20 minutes while controlling the temperature at 5°C. Next, a solution of potassium iodide (563 mg (3.391 mmol)) and iodine (419 mg (1.651 mmol)) in 7 ml of water was slowly added dropwise while controlling the temperature at 7°C or less. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with 10% sodium hydrogensulfite and dried over anhydrous sodium sulfate. The solvent was evaporated and the residue was purified by chromatography (dichloromethane: ethyl acetate = 8:2) to obtain 743 mg (58.6%) of the title compound as a yellowish brown amorphous substance.
$^1$H-NMR($CDCl_3$)δ:
7.68(d,J=7.08Hz,1H), 6.80-7.20(m,5H), 3.68-3.92(m,1H),
2.51-3.36(m,13H), 2.35(s,6H)
TLC (dichloromethane: ethyl acetate = 9:1); Rf = 0.33
FAB/MS (m/z) ; 463 ($MH^+$)

Example 17

(±)-trans-5-amino-2-hydroxy-3-[4-[5-iodo-2-methylphenyl]-piperazinyl]tetralin (DRF383)

(1) Synthesis of 1-[5-iodo-2-methylphenyl]piperazine

20 ml of n-buthanol and 1.0 g (4.291 mmol) of 5-iodo-2-methylaniline were added to 766 mg (4.292 mmol) of bis(2-chloroethyl)amine hydrochloride. The mixture was heated with stirring to dissolve bis(2-chloroethyl)- amine hydrochloride. 910 mg (8.686 mmol) of sodium carbonate was added to the reaction solution and the mixture was refluxed for 23 hours. After cooling, 2N sodium hydroxide was added and the mixture was extracted with a water/dichloromethane mixture. The organic layer was dried over anhydrous sodium sulfate and the solvent was evaporated. The residue was purified by chromatography (dichloromethane:methanol = 97:3 → dichloromethane:methanol = 9:1) and the solvent was evaporated to obtain 526 mg (40.5%) of the title compound as white crystals. TLC (dichloromethane:methanol = 95:5); Rf = 0.12

(2) Synthesis of (±)-trans-5-amino-2-hydroxy-3-[4-[5-iodo-2-methylphenyl]piperazinyl]tetralin

15 ml of ethanol was added to 526 mg (1.741 mmol) of 1-(5-iodo-2-methylphenyl)piperazine to dissolve the piperazine. 448 mg (1.724 mmol) of N-[trifluoroacetyl]-1-amino-5,8-dihydronaphthalene oxide was added to the reaction solution and the mixture was refluxed for 16 hours. After cooling the reaction solution, the solvent was removed by evaporation and the residue was dissolved in 6 ml of methanol. 10 ml of 2N sodium hydroxide was added to the solution and the mixture was stirred for 21 hours. The reaction solution was extracted with a water/dichloromethane mixture, the organic layer was dried over anhydrous sodium sulfate, and the solvent was evaporated. The residue was purified by flash chromatography (dichloromethane:methanol = 98:2) to obtain 186 mg (23.1%) of (±)-trans-5-amino-2-hydroxy-3-[4-[5-iodo-2-methylphenyl]piperazinyl]tetralin as a white amorphous low polar substance.
$^1$H-NMR($CDCl_3$) δ:
6.62-7.35(m,6H), 6.58(Br,2H), 2.63-4.01(m,14H),

2.25(s,3H)
TLC (dichloromethane:methanol = 97:3); Rf = 0.59

Example 18

(±)-trans-5-iodo-2-hydroxy-3-[4-[2-methoxyphenyl]-piperazinyl]tetralin (DRF400) and (±)-trans-5-iodo-2-hydroxy-3-[4-[2-hydroxyphenyl]-piperazinyl]tetralin (DRF403)

(1) Synthesis of (±)-trans-5-amino-2-hydroxy-3-[4-[2-methoxyphenyl]piperazinyl]tetralin

4.0g (20.805 mmol) of 1-(2-methoxyphenyl)piperazine was dissolved in 100 ml of ethanol. 5.4 g (20.995 mmol) of N-(trifluoroacetyl)-1-amino-5,8-dihydronaphthalene oxide was added to the reaction solution and the mixture was refluxed for 19 hours. After cooling the reaction solution, the solvent was removed by evaporation. 30 ml of methanol and 50 ml of 2N sodium hydroxide were added to the residue and the mixture was stirred for 18 hours. The reaction solution was extracted with a water/dichloromethane mixture, the organic layer was dried over anhydrous sodium sulfate, and the solvent was evaporated. The residue was purified by chromatography (dichloromethane:methanol = 98:2) and the solvent was evaporated to obtain 3.66 g (45.7%) of the title compound as a white amorphous substance.
TLC(dichloromethane:methanol = 98:2); Rf=0.36
$^1$H NMR($CDCl_3$)δ;
2.62-3.73(m,15H), 3.86(s,3H), 3.90-4.02(m,1H),
6.47-6.60(m,2H), 6.79-7.07(m,5H)
FAB/MS m/z; 354($MH^+$)

(2) Synthesis of (±)-trans-5-iodo-2-hydroxy-3-[4-[2-methoxyphenyl]piperazinyl]tetralin (DRF400)

23 ml of acetic acid and 15 ml of concentrated hydrochloric acid were added to 3.36 g (9.506 mmol) of (±)-trans-5-amino-2-hydroxy-3-[4-[2-methoxyphenyl] piperazinyl]tetralin and the mixture was stirred to cool with ice to a temperature of 5°C or less. 759 mg of sodium nitrite (11.000 mmol in 15 ml of water) was slowly added dropwise while maintaining the temperature of the reaction solution at 7°C. The reaction solution was stirred for 20 minutes while controlling the temperature at 5°C. Next, a solution of potassium iodide (1.95 g (11.746 mmol)) and iodine (1.4 g (5.516 mmol)) in 15 ml of water was slowly added dropwise while controlling the temperature at 7°C. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with a 10% sodium hydrogensulfite solution, and dried over anhydrous sodium sulfate. After removing the solvent by evaporation, the residue was purified by chromatography (dichloromethane:ethyl acetate = 9:1). The solvent was evaporated to obtain 1.55 g (35.2%) of the title compound as a yellow amorphous substance.
TLC (dichloromethane:methanol = 98:2); Rf = 0.50
$^1$H NMR($CDCl_3$)δ;
2.67-4.08(m,17H), 6.83-7.26(m,6H), 7.70(d,J=7.32Hz,1H)
FAB/MS m/z; 465 ($MH^+$)

(3) Synthesis of (±)-trans-5-iodo-2-hydroxy-3-[4-[2-hydroxyphenyl]piperazinyl]tetralin (DRF403)

35 ml of 47% hydrobromic acid was added to 1.4 g (3.015 mmol) of (±)-trans-5-iodo-2-hydroxy-3-[4-[2-methoxyphenyl]-piperazinyl]tetralin and the mixture was refluxed for 3 days. The reaction solution was cooled with ice and neutralized with the addition of 6N sodium hydroxide. The reaction solution was extracted with a water/dichloromethane mixture and the extract was dried over anhydrous sodium sulfate. After removing the solvent by evaporation, the residue was purified by chromatography (dichloromethane:methanol = 99:1). The solvent was evaporated to obtain 821 mg (60.5%) of the title compound as a light yellow amorphous substance.
TLC (dichloromethane:methanol = 99:1); Rf = 0.43
$^1$H NMR($CDCl_3$)δ;
2.68-4.00(m,15H), 6.75-7.25(m,6H), 7.71(d,J=7.56Hz,1H)
FAB/MS m/z; 451($MH^+$)

Example 19

(±)-trans-5-iodo-2-hydroxy-3-[4-[N-acetyl-2-aminophenyl]-piperazinyl]tetralin (DRF401) and (±)-trans-5-iodo-2-hydroxy-3-[4-[2-aminophenyl]-piperazinyl]tetralin (DRF402)

(1) Synthesis of N-benzyl-1-[2-nitrophenyl]-2,6-piperazinedione

48 ml of acetic acid anhydride was added to 12 g (53.756 mmol) of N-benzyliminodiacetic acid and the mixture was stirred for one hour at 150°C. The reaction solution was allowed to cool, followed by evaporation of the solvent. 96 ml of toluene and 7.42 g (53.718 mmol) of 2-nitroaniline were added to the residue, and the mixture was stirred for one hour at 100°C. The reaction solution was allowed to stand overnight and the solvent was evaporated. 60 ml of acetic acid anhydride was added to the residue and the mixture was stirred for 20 minutes at 140°C. After cooling and evaporating the solvent, the residue was purified by chromatography (dichloromethane:methanol = 98:2) and the solvent was evaporated to obtain 16.94 g (96.9%) of the title compound as brown crystals.
TLC (dichloromethane); Rf = 0.51
$^1$H NMR($CDCl_3$)δ;
3.36-3.86(m,6H), 7.26-7.76(m,8H), 8.16-8.27 (m, 1H)

(2) Synthesis of N-benzyl-1-[2-nitrophenyl]piperazine

16.94 g (52.072 mmol) of N-benzyl-1-[2-nitrophenyl]-2, 6-piperazinedione was dissolved in 50 ml of tetrahydrofuran. 20 ml of boron hydride was added to the solution and the mixture was refluxed for 13 hours in a nitrogen stream. 150 ml of 2.4 N hydrochloric acid solution and 50 ml of ethanol were slowly added to the reaction solution, and the mixture was stirred for two hours while heating at 80°C. The reaction solution was cooled, neutralized with 2N sodium hydroxide solution, and extracted with a water/dichloromethane mixture. The organic layer was dried over anhydrous sodium sulfate and the solvent was evaporated. The residue was purified by chromatography (dichloromethane → dichloromethane:methanol = 98:2) and the solvent was evaporated to obtain 9.7 g (62.7%) of the title compound as a brown oily substance.
TLC (dichloromethane:methanol = 99:1); Rf = 0.40
$^1$H NMR($CDCl_3$) δ;
2.55-2.65(m,4H), 3.03-3.13(m,4H), 3.57(s,2H),
6.91-.46(m,8H), 7.74(d,J=9.52Hz,1H)

(3) Synthesis of N-benzyl-1-[2-aminophenyl]piperazine

80 ml of 75% ethanol and 21 g of iron powder were added to 9.7 g (32.621 mmol) of N-benzyl-1-[2-nitrophenyl] piperazine. After the addition of 6 ml of concentrated hydrochloric acid, the mixture was refluxed for one hour. The reaction solution was cooled with ice, neutralized with 6N sodium hydroxide, and filtered through celite. The filtrate was extracted with a mixture of water and dichloromethane. The organic layer was dried over anhydrous sodium sulfate. After removing the solvent by evaporation, the residue was purified by chromatography (dichloromethane:methanol = 98:2). The solvent was evaporated to obtain 6.05 g (67.1%) of the title compound as a yellowish brown oily substance.
TLC (dichloromethane:methanol = 97:3); Rf = 0.61
$^1$H NMR($CDCl_3$)δ;
2.55-2,65(m,4H), 2.89-2.99(m,4H), 3.58(s,2H),
6.65-7.51(m,9H)

(4) Synthesis of N-benzyl-1-[N-acetyl-2-aminophenyl]-piperazine

40 ml of dichloromethane and 3.2 ml (23.085 mmol) of triethylamine were added to 6.05 g (22.628 mmol) of N-benzyl-1-[2-aminophenyl]piperazine and the mixture was stirred while cooling with ice. 1.7 ml(23.822 mmol) of acetyl chloride was slowly added to the solution, and the mixture was stirred for 30 minutes while cooling with ice. After removing the solvent by evaporation, the residue was purified by chromatography (dichloromethane:methanol = 98: 2). The solvent was evaporated to obtain 6.6 g (94.3%) of the title compound as a brown oily substance.
TLC (dichloromethane:methanol = 97:3); Rf = 0.51
$^1$H NMR($CDCl_3$)δ;
2.18(s,3H), 2.56-2,66(m,4H), 2.83-2.92(m,4H), 3.58(s,2H),
7.02-7.40(m,9H), 8.29-8.47(m,1H)

(5) Synthesis of 1-[N-acetyl-2-aminophenyl]piperazine

6.6 g (21.331 mmol) of N-benzyl-1-[N-acetyl-2-aminophenyl]piperazine was dissolved in 50 ml of methanol. 5.0 g of palladium-carbon was added to the solution to carry out catalytic reduction (17 hours). The reaction solution was filtered and the solvent was evaporated to obtain 3.45 g (73.7%) of the title compound as a light yellow oily substance.
TLC (dichloromethane:methanol = 95:5); Rf = 0.02
$^1$H NMR($CDCl_3$)δ;
2.21(s,3H), 2.85-3.28(m,9H), 7.10-7.28(m,4H),
8.24-8.44(m,1H)
FAB/MS m/z; 220($MH^+$)

(6) Synthesis of (±)-trans-5-amino-2-hydroxy-3-[4-[N-acetyl-2-aminophenyl]piperazinyl]tetralin

1.93 g (8.802 mmol) of 1-[N-acetyl-2-aminophenyl] piperazine was dissolved in 40 ml of ethanol. 2.3g (8.942 mmol) of N-(trifluoroacetyl)-1-amino-5,8-dihydronaphthalene was added to the reaction solution and the mixture was refluxed for 21 hours. After cooling the reaction solution, the solvent was removed by evaporation. 10 ml of methanol and 40 ml of 2N sodium hydroxide were added to the residue and the mixture was stirred for 24 hours. The reaction solution was extracted with a water/dichloromethane mixture. The organic layer was dried over anhydrous sodium sulfate and the solvent was evaporated. The residue was purified by chromatography (dichloromethane:ethyl acetate =9:1 → dichloromethane:methanol = 97:3). The solvent was evaporated to obtain 940 mg (28.1%) of the title compound as a light yellow amorphous substance.
TLC (dichloromethane:methanol = 97:3); Rf = 0.37
$^1$H NMR($CDCl_3$)δ;
2.23(s,3H), 2.64-4.04(m,16H), 6.48-7.27(m,7H),
8.32-8.41(m,1H)

(7) Synthesis of (+)-trans-5-iodo-2-hydroxy-3-[4-[N-acetyl-2-aminophenyl]piperazinyl]tetralin (DRF401)

12 ml of acetic acid and 6 ml of concentrated hydrochloric acid were added to 940 mg (2.471 mmol) of (±)-trans-5-amino-2-hydroxy-3-[4-[N-acetyl-2-aminophenyl]piperazinyl]tetralin and the mixture was stirred to cool with ice to a temperature of 5°C or less. 195 mg of sodium nitrite (2.826 mmol in 12 ml of water) was slowly added dropwise while maintaining the temperature of the reaction solution at 7°C. The reaction solution was stirred for 20 minutes while controlling the temperature at 5°C. Next, a solution of potassium iodide (506 mg (3.048 mmol)) and iodine (373 mg (1.470 mmol)) in 6 ml of water was slowly added dropwise while controlling the temperature at 7°C. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with a 10% sodium hydrogensulfite solution, and dried over anhydrous sodium sulfate. After removing the solvent by evaporation, the residue was purified by chromatography (dichloromethane → dichloromethane:methanol = 98:2). The solvent was evaporated to obtain 442 mg (36.5%) of the title compound as a yellowish brown amorphous substance.
TLC (dichloromethane:methanol = 97:3); Rf = 0.56
$^1$HNMR($CDCl_3$)δ;
2.26(s,3H), 2.51-4.05(m,14H), 6.75-7.26(m,6H),
7.71(d,J=7.56Hz,1H), 8.32-8.42(m,1H)
FAB/MS m/z; 492 ($MH^+$)

(8) Synthesis of (±)-trans-5-iodo-2-hydroxy-3-[4-[2-aminophenyl]piperazinyl]tetralin (DRF402)

30 ml of 6N hydrochloric acid was added to 756 mg (1.539 mmol) of (±)-trans-5-iodo-2-hydroxy-3-[4-[N-acetyl-2-aminophenyl]piperazinyl]tetralin and the mixture was refluxed for 2 hours. The reaction solution was cooled and neutralized with 2N sodium hydroxide. The solution was extracted with a water/dichloromethane mixture, the organic layer was dried over anhydrous sodium sulfate, and the solvent was evaporated. The residue was purified by chromatography (dichloromethane → dichloromethane:methanol = 98:2) and the solvent was evaporated to obtain 552 mg (79.8%) of the title compound as a brown amorphous substance.
TLC (dichloromethane:methanol = 97:3); Rf = 0.63
$^1$H NMR($CDCl_3$)δ;
2.64-3.98(m,16H), 6.68-7.27(m,6H), 7.71(d,J=7.52Hz,1H)

FAB/MS m/z; 450($MH^+$)

Example 20

(-)-trans-5-iodo-2-hydroxy-3-[4-[2-aminophenyl]piperazinyl] tetralin (DRF419) and (+)-trans-5-iodo-2-hydroxy-3-[4-[2-aminophenyl]piperazinyl] tetralin (DRF435)

(1) Synthesis of (-)-trans-5-iodo-2-α-methoxy-α-trifluoro-methylphenylacetoxy-3-[4-[N-acetyl-2-aminophenyl]-piperazinyl]tetralin and (+)-trans-5-iodo-2-α-methoxy-α-trifluoromethylphenylacetoxy-3-[4-[N-acetyl-2-aminophenyl] piperazinyl]tetralin

818 mg (1.665 mmol) of (±)-trans-5-iodo-2-hydroxy-3-[4-[N-acetyl-2-aminophenyl]piperazinyl]tetralin was dissolved in 15 ml of dichloromethane and 5 mg (0.041 mmol) of 4-dimethylaminopyridine and 231 μl (1.666 mmol) of triethylamine were added. The mixture was cooled to -80°C with stirring. 600 mg (2.375 mmol) of (-)-α-methoxy-α-trifluoromethylphenylacetylchloride was slowly added to the reaction solution. The reaction solution was stirred for 30 minutes at -80°C and 72 hours at room temperature. The reaction was terminated by adding water. The reaction solution was extracted with a water/dichloromethane mixture. The organic layer was dried over anhydrous sodium sulfate and the solvent was evaporated. The residue was purified by chromatography (dichloromethane:acetone =99:1) and the solvent was evaporated to obtain 307 mg (52.1%) of low polar (+)-trans-5-iodo-2-α-methoxy-α-trifluoromethylphenylacetoxy-3-[4-[N-acetyl-2-aminophenyl]piperazinyl]tetralin and 365 mg (62.0%) of high polar (-)-trans-5-iodo-2-α-methoxy-α-trifluoromethylphenylacetoxy-3-[4-[N-acetyl-2-aminophenyl]piperazinyl]tetralin.

- (+)-trans-5-iodo-2-α-methoxy-α-trifluoromethylphenyl-acetoxy-3-[4-[N-acetyl-2-aminophenyl]piperazinyl]tetralin
  TLC (dichloromethane:acetone = 99:1); Rf = 0.29
  $[\alpha]^{23}_D$ = +33.67 (C = 1.0, chloroform)
- (-)-trans-5-iodo-2-α-methoxy-α-trifluoromethylphenyl-acetoxy-3-[4-[N-acetyl-2-aminophenyl]piperazinyl]tetralin
  TLC (dichloromethane:acetone = 99:1); Rf = 0.24
  $[\alpha]^{23}_D$ = -12.5 (C = 1.0, chloroform)

(2) Synthesis of (-)-trans-5-iodo-2-hydroxy-3-[4-[N-acetyl-2-aminophenyl]piperazinyl]tetralin

356 mg (0.516 mmol)of (-)-trans-5-iodo-2-α-methoxy-α-trifluoromethylphenylacetoxy-3-[4-[N-acetyl-2-aminophenyl]piperazinyl]tetralin was dissolved in 2.0 ml of tetrahydrofuran and 1.0 ml of methanol. After the addition of 3.0 ml of 6N sodium hydroxide, the mixture was stirred for 72 hours at room temperature. The reaction solution was extracted with a water/dichloromethane mixture. The organic layer was dried over anhydrous sodium sulfate and the solvent was evaporated. The residue was purified by chromatography (dichloromethane:methanol = 98:2) and the solvent was evaporated to obtain 236 mg (92.1%) of the title compound as a light yellow amorphous substance.
$[\alpha]^{23}_D$ = -18.5 (C = 1.0, chloroform)

(3) Synthesis of (+)-trans-5-iodo-2-hydroxy-3-[4-[N-acetyl-2-aminophenyl]piperazinyl]tetralin

307 mg (0.434 mmol)of (+)-trans-5-iodo-2-α-methoxy-α-trifluoromethylphenylacetoxy-3-[4-[N-acetyl-2-aminophenyl] piperazinyl]tetralin was dissolved in 2.0 ml of tetrahydrofuran and 1.0 ml of methanol. After the addition of 3.0 ml of 6N sodium hydroxide, the mixture was stirred for 72 hours at room temperature. The reaction solution was extracted with a water/dichloromethane mixture. The organic layer was dried over anhydrous sodium sulfate and the solvent was evaporated. The residue was purified by chromatography (dichloromethane:methanol = 98:2) and the solvent was evaporated to obtain 206 mg (96.3%) of the title compound as a light yellow amorphous substance.
$[\alpha]^{23}_D$ = +40.83 (C = 1.0, chloroform)

(4) Synthesis of (-)-trans-5-iodo-2-hydroxy-3-[4-[2-aminophenyl]piperazinyl]tetralin (DRF419)

30 ml of 6N hydrochloric acid was added to 302 mg (0.615 mmol) of (-)-trans-5-iodo-2-hydroxy-3-[4-[N-acetyl-2-aminophenyl]piperazinyl]tetralin and the mixture was refluxed for 2 hours. The reaction solution was cooled and neutralized with 2N sodium hydroxide. The reaction solution was extracted with a mixture of water and dichloromethane. The organic layer was dried over anhydrous sodium sulfate and the solvent was evaporated. The residue was purified by chromatography (dichloromethane → dichloromethane:methanol = 98:2) and the solvent was evaporated to obtain 239 mg (86.8%) of the title compound as a brown amorphous substance.
$[\alpha]^{23}_D$ = -30.8 (C = 1.0, chloroform)

(5) Synthesis of (+)-trans-5-iodo-2-hydroxy-3-[4-[2-aminophenyl]piperazinyl]tetralin (DRF435)

30 ml of 6N hydrochloric acid was added to 206 mg (0.419 mmol) of (+)-trans-5-iodo-2-hydroxy-3-[4-[N-acetyl-2-aminophenyl]piperazinyl]tetralin and the mixture was refluxed for 2 hours. The reaction solution was cooled and neutralized with 2N sodium hydroxide. The reaction solution was extracted with a water/dichloromethane mixture. The organic layer was dried over anhydrous sodium sulfate and the solvent was evaporated. The residue was purified by chromatography (dichloromethane → dichloromethane:methanol = 98:2) and the solvent was evaporated to obtain 160 mg (85.1%) of the title compound as a brown amorphous substance.
$[\alpha]^{23}_{D}$ = +51.5 (C = 1.0, chloroform)

Example 21

(-)-trans-5-tributylstannyl-2-hydroxy-3-[4-[2-aminophenyl]-piperazinyl]tetralin (DRF434) and (+)-trans-5-tributylstannyl-2-hydroxy-3-[4-[2-aminophenyl]-piperazinyl]tetralin (DRF433)

(1) Synthesis of (-)-trans-5-tributylstannyl -2-hydroxy-3-[4-[2-aminophenyl]piperazinyl]tetralin (DRF434)

121 mg (0.269 mmol)of (-)-trans-5-iodo-2-hydroxy-3-[4-[2-aminophenyl]piperazinyl]tetralin was dissolved in 4 ml of toluene and nitrogen gas was bubbled through the mixture to deaerate. After the addition of 290 μl (0.574 mmol) of bis(tributyltin) and 19 mg (0.016 mmol) of tetrakistriphenylphosphine palladium, the reaction solution was refluxed for 22 hours in a nitrogen gas atmosphere. After removing the solvent by evaporation, the residue was purified by chromatography (n-hexane → dichloromethane → dichloromethane:methanol = 99:1). The solvent was evaporated to obtain 125 mg (75.8%) of the title compound as a brown oily substance.
TLC (dichloromethane:acetone = 98:2); Rf = 0.56
$^{1}$H NMR($CDCl_3$) δ ;
0.82-1.73(m,27H), 2.41-3.44(m,13H), 3.67-4.28(m,3H),
6.67-7.58(m, 7H)
FAB/MS m/z; 614($MH^+$)
$[\alpha]^{23}_{D}$ = -12.5 (C = 1.0, chloroform)

(2) Synthesis of (+)-trans-5-tributylstannyl-2-hydroxy-3-[4-[2-aminophenyl]piperazinyl]tetralin (DRF433)

101 mg (0.225 mmol) of (+)-trans-5-iodo-2-hydroxy-3-[4-[2-aminophenyl]piperazinyl]tetralin was dissolved in 3 ml of toluene and nitrogen gas was bubbled through the mixture to deaerate. After the addition of 240 μl (0.475 mmol) of bis(tributyltin) and 15 mg (0.013 mmol) of tetrakistriphenylphosphine palladium, the reaction solution was refluxed for 22 hours in a nitrogen gas atmosphere. After removing the solvent by evaporation, the residue was purified by chromatography (n-hexane → dichloromethane → dichloromethane:methanol = 99:1). The solvent was evaporated to obtain 90 mg (65.0%) of the title compound as a brown oily substance.
TLC (dichloromethane:acetone = 98:2); Rf = 0.48
FAB/MS m/z; 614($MH^+$)
$[\alpha]^{23}_{D}$ = +35.1 (C = 1.0, chloroform)

Example 22

(±)-trans-6-iodo-2-hydroxy-3-[4-[2-aminophenyl]piperazinyl] tetralin (DRG446)

(1) Synthesis 1-amino-2-nitro-5,8-dihydronaphthalene

11.87 g (51.111 mmol) of N-acetyl-1-amino-2-nitro-5,8-dihydronaphthalene was dissolved in 6N hydrochloric acid. The reaction solution was refluxed for 3.5 hours, then allowed to cool. After cooling, the reaction solution was alkalinized with the addition of 6N sodium hydroxide and extracted with dichloromethane. The organic layer was dried over anhydrous sodium sulfate and the solvent was evaporated. The residue was purified by chromatography (dichloromethane) and the solvent was evaporated to obtain 4.87 g (50.1%) of the title compound as a brown oily substance.
TLC (dichloromethane); Rf=0.54
$^{1}$H NMR($CDCl_3$)δ;
3.10-3.35(m,2H), 3.38-3.47(m,2H), 5.90(s,2H),
6.78(d,J=8.75Hz,1H), 7.98(d,J=9.03Hz,1H)
FAB/MS m/z; 191($MH^+$)

(2) Synthesis 2-nitro-5,8-dihydronaphthalene

60 ml of acetic acid and 30 ml of concentrated hydrochloric acid were added to 4.87 g (25.605 mmol)of 1-amino-2-nitro-5,8-dihydronaphthalene and the mixture was stirred to cool with ice to a temperature of 5°C or less. 1.9 mg of sodium nitrite (27.536 mmol in 10 ml of water) was slowly added dropwise while maintaining the temperature of the reaction solution at 7°C. The reaction solution was stirred for one hour while controlling the temperature at 5°C. 90 ml of 50% hypophosphorous acid was slowly added dropwise while maintaining the temperature of the reaction solution at 7°C. The reaction mixture was stirred for one hour while cooling with ice, then overnight at room temperature. The reaction solution was neutralized with the addition of 6N sodium hydroxide while cooling and extracted using a water/dichloromethane mixture. The organic layer was dried over anhydrous sodium sulfate. The solvent was evaporated and the residue was purified by chromatography (chloroform). The solvent was evaporated to obtain 3.6 g (89.6%) of the title compound as a yellowish brown oily substance.
TLC (dichloromethane); Rf=0.73
$^1$H NMR($CDCl_3$)δ;
3.45-3.47(m,4H), 5.93(s,2H), 7.19-7.29(m,1H),
7.92-8.04(m,2H)

(3) Synthesis 2-nitro-5,8-dihydronaphthalene oxide

3.6 g (20.549 mmol) of 2-nitro-5,8-dihydronaphthalene was dissolved in 90 ml of dichloromethane and stirred to cool with ice. 9.0 g (36.507 mmol) of m-chloroperbenzoic acid was slowly added to the reaction solution. The mixture was stirred for 24 hours. After the addition of 100 ml of 2N sodium hydroxide, the reaction solution was stirred for 10 minutes. An organic layer was extracted from the reaction solution and dried over anhydrous sodium sulfate. After removing the solvent by evaporation, the residue was purified by chromatography (dichloromethane). The solvent was evaporated to obtain 2.12 g (53.9%) of the title compound as light yellow crystals.
TLC (dichloromethane); Rf=0.62
$^1$HNMR($CDCl_3$)δ;
3.33-3.36(m,4H), 3.50-3.64(m,2H), 7.16-7.28(m,1H),
7.93-8.04(m,2H)
FAB/MS m/z; 192($MH^+$)

(4) Synthesis of (±)-trans-6-nitro-2-hydroxy-3-[4-[N-acetyl-2-aminophenyl]piperazinyl]tetralin

2.12 g (11.088 mmol) of 2-nitro-5,8-dihydronaphthalene oxide and 2.45 g (11.173 mmol) of 1-(N-acetyl-2-aminophenyl)-piperazine were dissolved in 50 ml of ethanol and the reaction solution was refluxed for 27 hours. The reaction solution was extracted with a water/dichloromethane mixture and the resulting organic layer was dried over anhydrous sodium sulfate. After removing the solvent by evaporation, the residue was purified by chromatography (dichloromethane → dichloromethane:methanol = 97:3). The solvent was evaporated to obtain 912 mg (20.0%) of the title compound as a yellow oily substance.
TLC (dichloromethane:methanol = 98:2); Rf = 0.39
FAB/MS m/z; 411($MH^+$)

(5) Synthesis of (+)-trans-6-amino-2-hydroxy-3-[4-[N-acetyl-2-aminophenyl]piperazinyl]tetralin

1.46 g of iron powder, 7.0 ml of 75% ethanol, and 400 μl of concentrated hydrochloric acid were added to 912 mg (2.222 mmol) of (±)-trans-6-nitro-2-hydroxy-3-[4-[N-acetyl-2-aminophenyl]piperazinyl]tetralin and the mixture was refluxed for one hour. The reaction mixture was allowed to cool to room temperature and filtered through celite. The filtrate was alkalinized with 2N sodium hydroxide. The reaction solution was extracted with a water/dichloromethane mixture. The organic layer was dried over anhydrous sodium sulfate. The solvent was evaporated. The residue was crystallized in ether to obtain 735 mg (87.0%) of yellow crystals.
TLC (dichloromethane:methanol = 97:3); Rf = 0.28
$^1$H NMR($CDCl_3$)δ:
2.23(s,3H), 2,62-3.35(m,13H), 3.61-3.91(m,1H),
6.46-6.55(m,2H), 6.87-7.26(m,5H), 8.31-8.44(m,1H)
FAB/MS m/z; 381($MH^+$)

(6) Synthesis of (+)-trans-6-iodo-2-hydroxy-3-[4-[N-acetyl-2-aminophenyl]piperazinyl]tetralin

13 ml of acetic acid and 7 ml of concentrated hydrochloric acid were added to 941 mg (2.473 mmol) of (±)-trans-6-amino-2-hydroxy-3-[4-[N-acetyl-2-aminophenyl]piperazinyl]tetralin and the mixture was stirred to cool with ice to a temperature of 5°C or less. 184 mg of sodium nitrite (2.667 mmol in 7 ml of water) was slowly added dropwise while maintaining the temperature of the reaction solution at 7°C. The reaction solution was stirred for 20 minutes while controlling the temperature at 5°C. Next, a solution of potassium iodide (495 mg (2.982 mmol)) and iodine (375 mg (1.477 mmol)) in 13 ml of water was slowly added dropwise while controlling the temperature at 7°C. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with a 10% sodium hydrogensulfite solution, and dried over anhydrous sodium sulfate. After removing the solvent by evaporation, the residue was purified by chromatography (chloroform:methanol = 97:3). The solvent was evaporated to obtain 425 mg (35.0%) of the title compound as a yellow amorphous substance.
TLC (dichloromethane:methanol = 97:3); Rf = 0.48
$^1$H NMR($CDCl_3$)δ:
2.23(s,3H), 2.72-3.52(m,13H), 3.81-4.04(m,1H),
6.82-7.50(m,7H), 8.34-8.41(m,1H)
FAB/MS m/z; 492($MH^+$)

(7) Synthesis of (±)-trans-6-iodo-2-hydroxy-3-[4-[2-aminophenyl]piperazinyl]tetralin (DRG446)

30 ml of 6N hydrochloric acid was added to 400 mg (0.814 mmol) of (±)-trans-6-iodo-2-hydroxy-3-[4-[N-acetyl-2-aminophenyl]piperazinyl]tetralin and the mixture was refluxed for 2 hours. The reaction solution was cooled and neutralized with 2N sodium hydroxide. The filtrate was extracted with a water/dichloromethane mixture, the organic layer was dried over anhydrous sodium sulfate, and the solvent was evaporated. The residue was purified by chromatography (dichloromethane → dichloromethane:methanol = 97:3) and the solvent was evaporated to obtain 141 mg (38.5%) of the title compound as a yellow amorphous substance.
TLC (dichloromethane:methanol = 97:3); Rf = 0.58
$^1$H NMR($CDCl_3$)δ;
2.60-3.41(m,13H), 3.65-3.94(m,1H), 6.69-7.09(m,5H),
7.41-7.48(m,2H)
FAB/MS m/z; 450($MH^+$)

Example 23

(-)-trans-5-tributylstannyl-2-hydroxy-3-(4-phenyl-1-piperazinyl)tetralin (DRC136)

(1) Synthesis of (-)-trans-5-tributylstannyl-2-hydroxy-3-(4-phenyl-1-piperazinyl)tetralin (DRC136)

66 mg (0.152 mmol) of (-)-trans-5-iodo-2-hydroxy-3-(4-phenyl-1-piperazinyl)tetralin was dissolved in 2.5 ml of tetrahydrofuran and the solution was cooled to -78°C. 0.21 ml (0.338 mmol) of 1.6 M n-butyl lithium was slowly added to the solution, and the mixture was stirred for 1.5 hours at -78°C. 110 mg (0.388 mmol) of tributyltin chloride was added to the reaction solution and the mixture was stirred for 12 hours at room temperature. The reaction was terminated by adding 20 ml of a 5% ammonium chloride solution and extracted with a mixture of water and dichloromethane. The organic layer was dried over anhydrous sodium sulfate and the solvent was removed by evaporation. The residue was purified by chromatography (dichloromethane → n-hexane:ethyl acetate = 7:3). The solvent was evaporated to obtain 25 mg (27.5%) of the title compound as a light yellow oily substance.
$^1$H-NMR($CDCl_3$)δ:
0.94-0.84(m,9H), 1.13-1.01(m,3H), 1.42-1.29(m,9H),
1.63-1.43(m,9H), 2.73-2.68(m,2H), 2.89-2.83(m,4H),
2.99-2.95(m,2H), 3.35-3.22(m,5H),
3.95-3.89(m,1H),4.16(s,1H), 6.89(t,J=6.83Hz,1H),
6.97(d,J=8.30Hz,2H), 7.13-7.07(m,2H), 7.31-7.24(m,3H)
FAB/MS m/z:597($MH^+$)

Example 24

(±)-trans-7-iodo-2-hydroxy-3-[4-phenylpiperazinyl]tetralin (DRG449)

(1) Synthesis of (±)-trans-N-acetyl-8-amino-7-nitro-2-hydroxy-3-[4-phenylpiperazinyl]tetralin

4.0 g (16.113 mmol) of N-acetyl-1-amino-2-nitro-5,8-dihydronaphthalene oxide was dissolved in ethanol. After the addition of 2.72 g (16.766 mmol) of 1-phenylpiperazine, the mixture was refluxed for 20 hours. The reaction solution was cooled to room temperature and the solvent was evaporated. The residue was purified by chromatography (dichloromethane:methanol = 99:1) and the solvent was evaporated to obtain 1.387 g (21.0%) of (±)-trans-N-acetyl-5-amino-6-nitro-2-hydroxy-3-[4-phenylpiperazinyl]tetralin and 0.984 g (14.9%) of (±)-trans-N-acetyl-8-amino-7-nitro-2-hydroxy-3-[4-phenylpiperazinyl]tetralin, both as yellow crystals.
TLC (dichloromethane:methanol = 97:3); Rf = 0.38
$^1$H NMR ($CDCl_3$) δ;
2.23(s,3H), 2.58-3.48(m,13H), 3.76-3.99(m,1H),
6.82-7.29(m,7H), 7.81(d, J=8.06Hz,1H), 8.23(s,1H)
FAB/MS m/z: 411 ($MH^+$)
m.p = 216.1-218.7°C

(2) Synthesis of (±)-trans-8-amino-7-nitro-2-hydroxy-3-[4-phenylpiperazinyl]tetralin

100 ml of 6N hydrochloric acid was added 1.892 g (4.609 mmol) of (±)-trans-N-acetyl-8-amino-7-nitro-2-hydroxy-3-[4-phenylpiperazinyl]tetralin. The mixture was refluxed for four hours. After cooling, the reaction solution was alkalinized with the addition of 2N sodium hydroxide and extracted with a water/dichloromethane mixture. The organic layer was dried over anhydrous sodium sulfate and the solvent was removed by evaporation. The residue was purified by chromatography (dichloromethane:ethyl acetate = 7:3). The solvent was evaporated to obtain 1.253 g (61.94%) of the title compound as yellow crystals.
TLC (dichloromethane:methanol = 95:5); Rf = 0.46
$^1$H NMR($CDCl_3$) δ;
2.57-4.00(m,14H), 6.29-6.60(m,3H), 6.89-6.90(m,3H),
7.21-7.38(m,2H), 7.98(d,J=8.79Hz,1H)
m.p = 229.9-232.8°C

(3) Synthesis of (±)-trans-7-nitro-2-hydroxy-3-[4-phenylpiperazinyl]tetralin

18 ml of acetic acid and 9 ml of concentrated sulfuric acid were added to 1.253 g (3.401 mmol) of (±)-trans-8-amino-7-nitro-2-hydroxy-3-[4-phenylpiperazinyl]tetralin and the mixture was stirred while cooling with ice. 260 mg of sodium nitrite (3.768 mmol in 9 ml of water) was slowly added dropwise while maintaining the temperature of the reaction solution at 10°C. After stirring for one hour at 5°C or less, 12 ml of 50% hypophosphorous acid was added and the mixture was stirred for 17 hours at room temperature. The reaction solution was neutralized with a saturated sodium hydrogencarbonate solution and extracted with a water/dichloromethane mixture. The organic layer was dried over anhydrous sodium sulfate and the solvent was removed by evaporation. The residue was purified by chromatography (dichloromethane:ethyl acetate = 8:2). The solvent was evaporated to obtain 0.779 g (64.8%)of the title compound as light yellow crystals.
TLC (dichloromethane:methanol = 97:3); Rf = 0.65
$^1$H NMR($CDCl_3$) δ ;
2.59-4.00(m,14H), 6.80-6.99(m,3H), 7.29-7.37(m,3H),
7.93-7.99(m,2H)
FAB/MS m/z: 354($MH^+$)
m.p = 183.6-184.6°C

(4) Synthesis of (±)-trans-7-amino-2-hydroxy-3-[4-phenylpiperazinyl]tetralin

9 ml of 75% ethanol and 4.63 g of iron powder were added to 807 mg (2.283 mmol) of (±)-trans-7-nitro-2-hydroxy-3-[4-phenylpiperazinyl]tetralin. 450 µl of concentrated hydrochloric acid was added and the mixture was refluxed for one hour. The reaction suspension was neutralized with 2N sodium hydroxide and filtered through celite. The filtrate was extracted with a water/dichloromethane mixture. The organic layer was dried over anhydrous sodium sulfate and the solvent was removed by evaporation. The residue was purified by chromatography (dichloromethane:

methanol= 95:5). The solvent was evaporated to obtain 530 mg (71.8%) of the title compound as red crystals.
TLC (dichloromethane:methanol = 95:5); Rf = 0.44
$^1$H NMR($CDCl_3$) δ ;
2.58-4.00(m,14H), 6.42-6.59(m,2H), 6.69-6.96(m,4H), 7.11-7.36(m,2H)
FAB/MS m/z: 324($MH^+$)
m.p = 151.8-152.3°C

(5) Synthesis of (±)-trans-7-iodo-2-hydroxy-3-[4-phenylpiperazinyl]tetralin (DRG449)

9 ml of acetic acid and 4 ml of concentrated hydrochloric acid were added to 526 mg (1.626 mmol) of (±)-trans-7-amino-2-hydroxy-3-[4-phenylpiperazinyl]tetralin and the mixture was stirred while cooling with ice. 120 mg of sodium nitrite (1.739 mmol in 9 ml of water) was slowly added dropwise while maintaining the temperature of the reaction solution at 10°C. The reaction solution was stirred for 20 minutes at 10°C or less. A solution of potassium iodide (325 mg (1.985 mmol)) and iodine (247 mg (0.976 mmol)) in 6 ml of water was slowly added dropwise while controlling the temperature at 10°C or less. The reaction mixture was stirred for 3 hours at 10°C or less and 16 hours at room temperature. The reaction solution was neutralized with a saturated sodium hydrogencarbonate solution and extracted with a water/dichloromethane mixture. The resulting organic layer was washed with 10% sodium sulfite. The organic layer was dried over anhydrous sodium sulfate and the solvent was removed by evaporation. The residue was purified by chromatography (dichloromethane:ethyl acetate = 9:1). The solvent was evaporated to obtain 287 mg (40.7%) of the title compound as skin color crystals.
TLC (dichloromethane:methanol = 95:5); Rf = 0.8
$^1$H NMR($CDCl_3$)δ;
2.57-3.05(m,8H), 3.15-3.58(m,5H), 3.71-4.00(m,1H), 6.77-6.97(m,4H), 7.11-7.47(m,4H)
FAB/MS m/z: 435($MH^+$)
m.p=196.6-198.0°C

Example 25

(±)-trans-5-iodo-2-hydroxy-3-[4-[2-aminomethylphenyl]-piperazinyl]tetralin

(1) Synthesis of N-benzyl-1-[2-cyanophenyl]piperazine

10 g (54.939 mmol) of 2-bromobenzonitrile was dissolved in 25 g (141.836 mmol) of 1-benzylpiperazine and the solution was refluxed for 21 hours. The reaction solution was purified by chromatography (dichloromethane → dichloromethane:methanol = 98:2) and the solvent was evaporated to obtain 4.81 g (31.6%) of the title compound as a yellowish brown oily substance.
TLC (dichloromethane:methanol = 99:1); Rf = 0.28
$^1$H NMR($CDCl_3$)δ ;
2.62-2.72(m,4H), 3.19-3.28(m,4H), 3.59(s,2H), 6.94-7.05(m,2H), 7.25-7.58(m,7H)

(2) Synthesis of N-benzyl-1-[2-aminomethylphenyl]piperazine

50 ml of tetrahydrofuran was slowly added to 3.5 g (92.227 mmol) of lithium aluminum hydride to obtain a suspension. A solution of 4.59 g (16.548 mmol) of N-benzyl-1-[2-cyanophenyl] piperazine in 20 ml of tetrahydrofuran was slowly added dropwise to the suspension. The reaction solution was refluxed for one hour. After cooling, water was carefully added. The reaction solution was filtered through celite and the filtrate was extracted with a water/dichloromethane mixture. The organic layer was dried over anhydrous sodium sulfate, and the solvent was evaporated to obtain 4.05 g (86.9%) of the title compound as a yellow oily substance.
TLC (dichloromethane:methanol = 99:1); Rf = 0.00
$^1$H NMR($CDCl_3$) δ;
1.85(Br,2H), 2.56-2.66(m,4H), 2.91-3.01(m,4H), 3.58(s,2H), 3.87(s,2H), 7.14-7.33(m,9H)

(3) Synthesis of N-benzyl-1-[2-[N-acetyl-aminomethyl]-phenyl]piperazine

4.05 g (14.392 mmol) of N-benzyl-1-[2-aminomethyl-phenyl]piperazine was dissolved in 30 ml of dichloromethane and 2.0 ml (14.428 mmol) of triethylamine was added to the solution. 1.1 ml (15.414 mmol) of acetyl chloride was slowly added dropwise to the solution with stirring while cooling with ice. The mixture was stirred for a further 30 minutes while cooling with ice. The reaction solution was filtered and solvent was evaporated from the filtrate. The residue was purified by chromatography (chloroform:acetone = 6:4). The solvent was evaporated to obtain 3.45 g (74.2%) of the title compound as a yellow oily substance.
TLC (dichloromethane:methanol = 97:3); Rf = 0.39
$^1$H NMR($CDCl_3$) δ ;
1.98(s,3H), 2.58-2.67(m,4H), 2.91-2.99(m,4H), 3.61(s,2H),
4.49(d, J=5.60Hz,2H), 7.09-7.33(m,9H)

(4) Synthesis of 1-[2-[N-acetyl-aminomethyl]phenyl]-piperazine

3.45 g (10.667 mmol) of N-benzyl-1-[2-[N-acetyl-aminomethyl]phenyl]piperazine was dissolved in 50 ml of methanol. 1.6 g of 10% palladium on carbon was added to the solution to carry out catalytic reduction in a hydrogen stream. The reaction solution was filtered and the solvent was evaporated to obtain 1.77 g (71.1%) of the title compound as a yellowish brown oily substance.
TLC (dichloromethane:methanol = 97:3); Rf = 0.00
$^1$H NMR($CDCl_3$) δ ;
1.99(s,3H), 3.01-3.09(m,8H), 4.47(d,J=5.37Hz,2H),
5.80(Br,1H), 7.13-7.35(m,4H)
FAB/MS m/z: 234($MH^+$)

(5) Synthesis of (±)-trans-5-amino-2-hydroxy-3-[4-[2-[N-acetyl-aminomethyl]phenyl]piperazinyl]tetralin

1.77 g (7.586 mmol) of 1-[2-[N-acetyl-aminomethyl]-phenyl]piperazine was dissolved in 35 ml of ethanol. 5.4 g (20.995 mmol) of N-(trifluoroacetyl)-1-amino-5, 8-dihydronaphthalene oxide was added to the reaction solution and the mixture was refluxed for 19 hours. After cooling the reaction solution, the solvent was removed by evaporation. 30 ml of methanol and 30 ml of 2N sodium hydroxide were added to the residue and the mixture was stirred for 18 hours. The reaction solution was extracted with a water/dichloromethane mixture. The organic layer was dried over anhydrous sodium sulfate and the solvent was evaporated. The residue was purified by chromatography (chloroform:methanol = 96:4) and the solvent was evaporated to obtain 1.58 g (52.8%) of the title compound as a brown amorphous substance.
TLC (dichloromethane:methanol = 96:4); Rf = 0.33
$^1$H NMR($CDCl_3$) δ ;
2.05(s,3H), 2.46-4.00(m,14H), 4.57(d,J=7.84Hz,2H),
6.16(Br,1H), 6.52-6.61(m,2H), 6.92-7.31(m,5H)

(6) Synthesis of (+)-trans-5-iodo-2-hydroxy-3-[4-[2-[N-acetyl-aminomethyl]phenyl]piperazinyl]tetralin

20 ml of acetic acid and 10 ml of concentrated hydrochloric acid were added to 1.58 g (4.005 mmol) of (±)-trans-5-amino-2-hydroxy-3-[4- [2-[N-acetyl-aminomethyl]phenyl]piperazinyl]tetralin and the mixture was stirred to cool with ice to a temperature of 5°C or less. 292 mg of sodium nitrite (4.232 mmol in 10 ml of water) was slowly added dropwise while maintaining the temperature of the reaction solution at 7°C or less. The reaction solution was stirred for 20 minutes while controlling the temperature at 5°C. Next, a solution of potassium iodide (800 mg (4.819 mmol)) and iodine (615 mg (2.423 mmol)) in 20 ml of water was slowly added dropwise while controlling the temperature at 7°C. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with a 10% sodium hydrogensulfite solution, and dried over anhydrous sodium sulfate. After removing the solvent by evaporation, the residue was purified by chromatography (dichloromethane:methanol = 97:3). The solvent was evaporated to obtain 1.07 g (53.0%) of the title compound as a brown amorphous substance.
TLC (dichloromethane:methanol = 97:3); Rf = 0.33
$^1$H NMR($CDCl_3$) δ ;
2.04(s,3H), 2,61-4.00(m,14H), 4.56(d,J=5.62Hz,2H),
6.26(Br,1H), 6.83-7.26(m,7H), 7.67-7.76(m,1H)
FAB/MS m/z; 506($MH^+$)

(7) Synthesis of (±)-trans-5-iodo-2-hydroxy-3-[4-[2-aminomethylphenyl]piperazinyl]tetralin

80 ml of 6N hydrochloric acid was added to 1.07 g (2.117 mmol) of (±)-trans-5-iodo-2-hydroxy-3-[4-[2-[N-acetyl-aminomethyl]phenyl]piperazinyl]tetralin and the mixture was refluxed for 2 hours. The reaction solution was cooled and neutralized with 2N sodium hydroxide. The solution was extracted with a water/dichloromethane mixture, the organic layer was dried over anhydrous sodium sulfate, and the solvent was evaporated. The residue was purified by chromatography (dichloromethane → dichloromethane:methanol = 8:2) and the solvent was evaporated to obtain 587 mg (59.8%) of the title compound as a brown amorphous substance.
TLC (dichloromethane:methanol = 9:1); Rf = 0.23
$^1$H NMR($CDCl_3$) δ ;
2,61-3.51(m,13H), 3.81-3.90(m,1H), 3.93(s,2H),
6.81-7.34(m,6H), 7.69(d,J=7.57Hz,1H)
FAB/MS m/z; 464($MH^+$)

Example 26

·(±)-trans-5-iodo-2-hydroxy-3-[4-[2-hydroxymethylphenyl] piperazinyl]tetralin

(1) Synthesis of N-benzyl-1-[2-formylphenyl]piperazine

3.95 g (14.241 mmol) of N-benzyl-1-[2-cyanophenyl]-piperazine was dissolved in 50 ml of dichloromethane and the solution was stirred in an argon gas stream with stirring. 65 ml of 1M diisobutyl aluminum hydride in n-hexane solution was slowly added to the reaction solution, and the mixture was stirred for 3.5 hours while cooling with ice. After terminating the reaction by slowly adding water, the reaction solution was filtered through celite. The filtrate was extracted with a mixture of water and dichloromethane, the organic layer was dried over anhydrous sodium sulfate, and the solvent was evaporated. The residue was purified by chromatography (chloroform:methanol = 97:3) and the solvent was evaporated to obtain 1.45 g (35.3%) of the title compound as a brown oily substance.
TLC (dichloromethane:methanol = 97:3); Rf = 0.59
$^1$H NMR($CDCl_3$)δ;
2,61-2.71(m,4H), 3.07-3.17(m,4H), 3.60(s,2H),
7.06-7.51(m,8H), 7.76-7.85(m,1H), 10.33(s,1H)

(2) Synthesis of N-benzyl-1-[2-hydroxymethylphenyl]-piperazine

40 ml of tetrahydrofuran was slowly added to 3. 0 g (79.051 mmol) of lithium aluminum hydride. A solution of 1.41 g (5.029 mmol) of N-benzyl-1-[2-formylphenyl]piperazine in 35 ml of tetrahydrofuran was slowly added to the suspenstion, followed by refluxing for one hour. After cooling, water was carefully added. The reaction solution was filtered through celite and extracted with a water/dichloromethane mixture. The organic layer was dried over anhydrous sodium sulfate and the solvent was evaporated. The residue was purified by chromatography (dichloromethane:methanol = 97:3) and the solvent was evaporated to obtain 1.05 g (73.9%) of the title compound as a brown oily substance.
TLC (dichloromethane:methanol = 97:3); Rf = 0.29
$^1$H NMR($CDCl_3$) δ ;
2.59-2.69(m,4H), 2.96-3.06(m,4H), 3.58(s,2H), 4.79(s,2H),
7.12-7.32(m,9H)

(3) Synthesis of 1-[2-hydroxymethylphenyl]piperazine

1.04 g (3.683 mmol) of N-benzyl-1-[2-hydroxymethyl-phenyl]piperazine was dissolved in 30 ml of methanol. 500 mg of 10% palladium on carbon was added to the solution to carry out catalytic reduction in a hydrogen stream. The reaction solution was filtered and the solvent was evaporated to obtain 547 mg (77.3%) of the title compound as a light yellow oily substance.
TLC (dichloromethane:methanol = 97:3); Rf = 0.00
$^1$H NMR($CDCl_3$)δ;
3.03-3.05(m,8H), 4.80(s,2H), 7.17-7.23(m,4H)
FAB/MS m/z: 193($MH^+$)

(4) Synthesis of (±)-trans-5-amino-2-hydroxy-3-[4-[2-hydroxymethylphenyl]piperazinyl]tetralin

547 mg (2.845 mmol) of 1-[2-hydroxymethylphenyl]-piperazine was dissolved in 13 ml of ethanol. 732 mg (2.846 mmol) of N-(trifluoroacetyl)-1- amino-5,8-dihydronaphthalene oxide was added to the reaction solution and the mixture was refluxed for 26 hours. After cooling the reaction solution, the solvent was removed by evaporation. 5 ml of methanol and 10 ml of 2N sodium hydroxide were added to the residue and the mixture was stirred for 18 hours. The reaction solution was extracted with a water/dichloromethane mixture, the organic layer was dried over anhydrous sodium sulfate, and the solvent was evaporated. The residue was purified by chromatography (dichloromethane:methanol = 98:2) and the solvent was evaporated to obtain 230 mg (22.8%) of the title compound as a white amorphous substance.
TLC (dichloromethane:methanol = 98:2); Rf = 0.25
$^1$H NMR($CDCl_3$) δ ;
2.64-3.21(m,10H), 3.26-3.98(m,5H), 4.83(s,2H),
6.51-6.59(m,3H), 6.71-7.31(m,6H)
FAB/MS m/z: 354 ($MH^+$)

(5) Synthesis of (±)-trans-5-iodo-2-hydroxy-3-[4-[2-hydroxymethylphenyl]piperazinyl]tetralin

4 ml of acetic acid and 2 ml of concentrated hydrochloric acid were added to 230 mg (0.651 mmol) of (±)-trans-5-amino-2-hydroxy-3-[4-[2-hydroxymethylphenyl]piperazinyl]tetralin and the mixture was stirred to cool with ice to a temperature of 5°C or less. 48 mg of sodium nitrite (0.696 mmol in 2 ml of water) was slowly added dropwise while maintaining the temperature of the reaction solution at 7°C. The reaction solution was stirred for 20 minutes while controlling the temperature at 5°C. Next, a solution of potassium iodide (130 mg (0.783 mmol)) and iodine (100 mg (0.394 mmol)) in 4 ml of water was slowly added dropwise while controlling the temperature at 7°C. The reaction solution was stirred for 3.5 hours while controlling the temperature at 5°C, followed by further stirring overnight at room temperature. The reaction solution was neutralized with the addition of 6N sodium hydroxide while cooling and extracted using a water/dichloromethane mixture. The organic layer was washed with a 10% sodium hydrogensulfite solution, and dried over anhydrous sodium sulfate. After evaporating the solvent, the residue was purified by chromatography (dichloromethane:methanol = 98:2). The solvent was evaporated to obtain 183 mg (60.6%) of the title compound as a white amorphous substance.
TLC (dichloromethane:methanol = 98:2); Rf = 0.35
$^1$H NMR($CDCl_3$) δ ;
2.61-3.19(m,10H), 3.32-3.98(m,5H), 4.84(s,2H),
6.74-7.33(m,6H), 7.69(d,J=7.08Hz,1H)
FAB/MS m/z; 465($MH^+$)

Example 27

Compounds listed in the column for Product Compounds in Table 1 were prepared by using the compounds in the column for Raw Materials in Table 1 as the compound (II) of the above-described Synthesis Processs A. The lower line compounds in the column for Product Compounds are compounds obtained by reducing the carbonyl group of the upper line compounds.

Table 1

| No. | Raw Material | Product Compound |
|---|---|---|
| 1 | 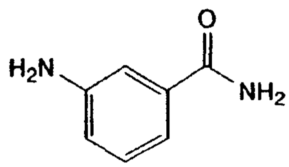 | 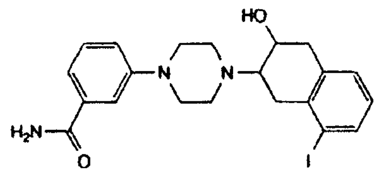 |
| 2 | | 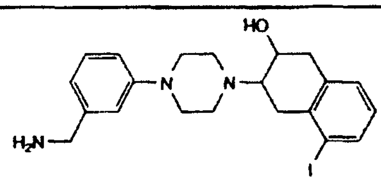 |
| 3 | 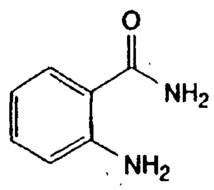 | 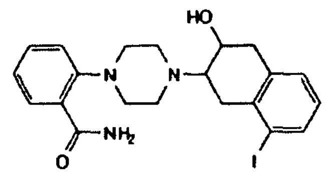 |
| 4 | | 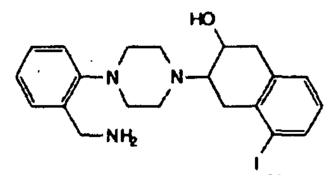 |
| 5 | 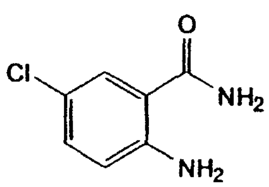 | 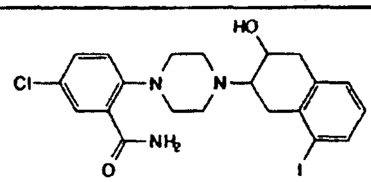 |
| 6 | | 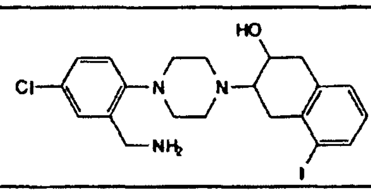 |

Example 28

Compounds listed in the column for Product Compounds in Table 2 were prepared by using the compounds in the column for Raw Materials in Table 2 as the compound (II) of the above-described Synthesis Processs A. The lower line compounds in the column for Product Compounds are compounds obtained by converting the alkoxyl group of the upper line compounds into a hydroxyl group.

Table 2

| No. | Raw Material | Product Compound |
|---|---|---|
| 1 | 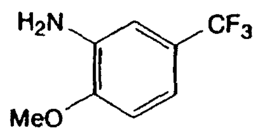 | 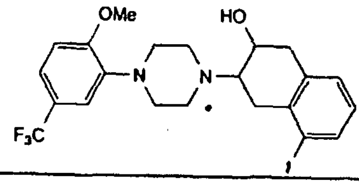 |
| 2 | | 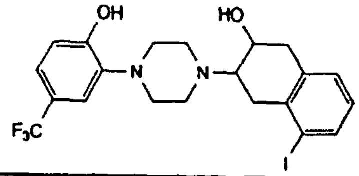 |

Example 29

Compounds listed in the column for Product Compounds in Table 3 were prepared by using the compounds in the column for Raw Materials in Table 3 as the compound (II) of the above-described Synthesis Processs C.

Table 3

| No. | Raw Material | Product Compound |
|---|---|---|
| 1 | 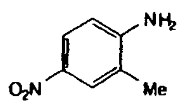 | 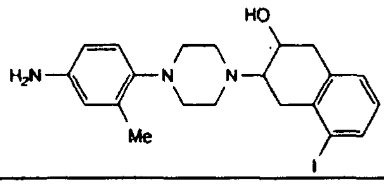 |
| 2 | 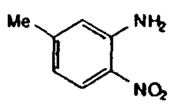 | 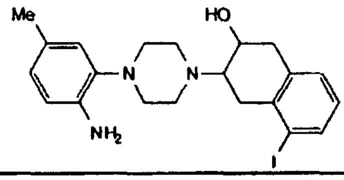 |
| 3 | 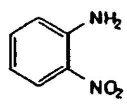 | 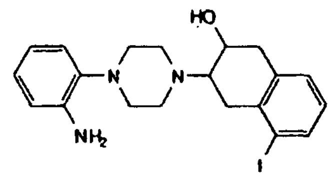 |
| 4 | 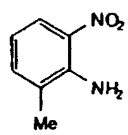 | 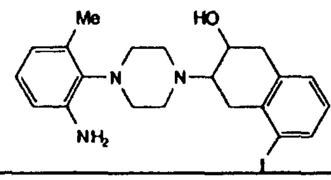 |
| 5 | 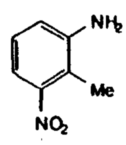 | 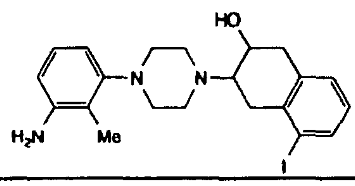 |
| 6 | 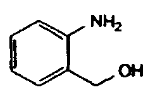 | 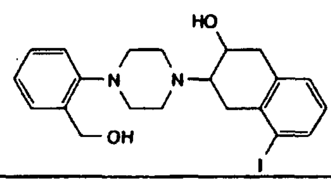 |

Example 30

Compounds listed in the column for Product Compounds in Table 4 were prepared by using the compounds in the column for Raw Materials in Table 4 as the compound (II) of the above-described Synthesis Processs C. The lower line compounds in the column for Product Compounds are compounds obtained by converting the arkoxyl group of the upper line compounds into a hydroxyl group.

Table 4

| No. | Raw Material | Product Compound |
|---|---|---|
| 1 | 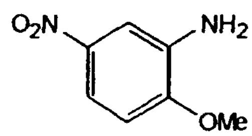 | 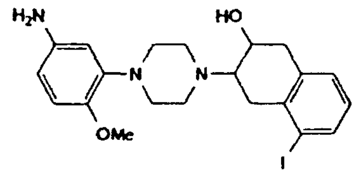 |
| 2 | | 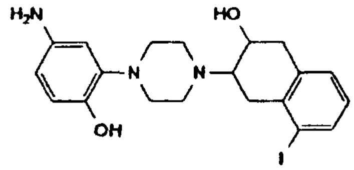 |
| 3 | 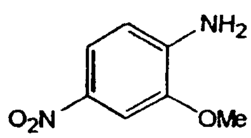 | 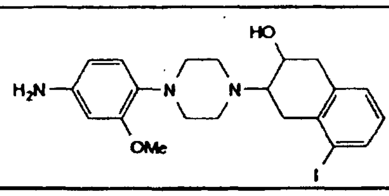 |
| 4 | | 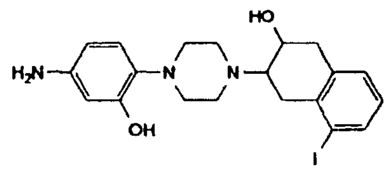 |

Example 31

Receptor binding test:

Properties relating to binding of the vesamicol piperazine derivatives of the present invention to various receptors were examined as follows. Specifically, ligands which combine with each receptor were incubated together with homogenates prepared as follows.

To measure affinity of vesamicol receptors, I-125 labeled 5-iodobenzo vesamicol (synthesized and labeled according to Jung et al., J. Med. Chem. 33, 2065-2068, 1990) was added to a brain homogenate prepared as follows. The vesamicol piperazine derivatives synthesized in the Examples were added at various concentrations. The mixture was then incubated for three hours at 37°C.

To measure affinity to adrenaline $\alpha_1$ receptors, tritium-labeled prazosin was added to a brain homogenate prepared as follows, the vesamicol piperazine derivatives synthesized in the Examples were added at various concentrations, and the mixture was incubated for 30 minutes at 37°C. To measure affinity to sigma receptors, tritium-labeled (+) pentazocine was added to a brain homogenate prepared as follows. The vesamicol piperazine derivatives synthesized in the Examples were added at various concentrations, and the mixture was incubated for two hours at room temperature.

To measure affinity to serotonin 5-$HT_{1A}$ receptors, tritium-labeled 8-hydroxy-DPAT was added to a brain homogenate prepared as follows. The vesamicol piperazine derivatives synthesized in the Examples were added at various concentrations, and the mixture was incubated for 30 minutes at 37°C. To measure affinity to serotonin 5-$HT_{2A}$ receptors, tritium-labeled ketanserin was added to a brain homogenate prepared as follows. The vesamicol piperazine derivatives synthesized in the Examples were added at various concentrations, and the mixture was incubated for 15 minutes at 37°C.

Assuming that the specific binding of a radioactive ligand without vesamicol derivatives is 100%, the concentration of a vesamicol derivative, at which up to 50% of the specific binding of a radioactive ligand ($IC_{50}$) is inhibited, is calculated for each receptor using the following formula.

$$B\ (\%) = \frac{1\,0\,0 \times [U]^{\cap}}{[I\,C_{50}]^{\cap} + [U]^{\cap}}$$

wherein B(%) is the proportion of specific binding in the total binding, n indicates a Hill coefficient, and U is the concentration of unbound ligand.

(Preparation of samples for the measurement of vesamicol receptor activity)

Male Wistar rats were anesthetized with ether and were decapitated. The brains were immediately extracted. The cerebella were removed from the brains. 0.32 M sucrose (containing 4 mM HEPES (pH 7.4)) in an amount of five times the wet amount (weight) of this sample was added. The sample was homogenized by a potter-type homogenizer for 12 strokes while cooling with ice. 0.32 M sucrose in the same amount as the homogenate (total: 10 times the wet amount of the original sample) was added. The suspension thus obtained was centrifuged for 5 minutes at 1,000 x g. The precipitate was removed. The supernatant liquid was centrifuged at 9,200 x g for 15 minutes to separate the precipitate from the supernatant liquid. P2 fractions were suspended in a Tris-HCl buffer (pH 7.4) for binding experiments and stored at -80°C until the start of the binding experiment.

(Preparation of samples for measuring activity of adrenaline $\alpha 1$ receptor)

Male Wistar rats were anesthetized with ether and were decapitated. The brains were immediately extracted and the cerebral membranes were divided. 50 mM Tris-HCl/10 mM $MgCl_2$ buffer solution (pH 7.4) in the amount of 30 times the wet amount of the original sample was added to prepare a homogenate. The homogenate was centrifuged at 48,000 g for 30 minutes and the resulting pellets were suspended in the same buffer solution. This procedure was repeated twice. The pellets finally obtained were suspended in the buffer solution of approximately 10 times the amount of the pellets and stored at -80°C until the start of the experiment.

(Preparation of samples for the measurement of sigma receptor activity)

A male guinea pig was anesthetized with ether and was decapitated. The brain was immediately extracted. The cerebellum was removed from the brain. 10 mM Tris-sucrose buffer solution (0.32 M sucrose + 10 mM Tris-HCl (pH 7.4)) was added in the amount of 10 ml/g of the tissue. The mixture was homogenized by a potter-type homogenizer for 6-10 strokes. The homogenate was centrifuged at 1,000 x g for 10 minutes at 4°C and the supernatant liquid was collected. The resulting pellets were suspended in the Tris-sucrose buffer solution at the concentration of 2 ml/g, followed by centrifugation at 1,000 x g for 10 minutes at 4°C. The supernatant liquids obtained by the two centrifugation at 1,000 x g were combined and centrifuged at 31,000 x g for 15 minutes at 4°C. The resulting pellets were suspended in a 10 mM Tris-HCl buffer solution (pH 7.4) at a concentration of 3 ml/g of tissue and incubated for 30 minutes at 25°C. After incubation, the suspension was centrifuged at 31,000 x g for 15 minutes at 4°C. The pellets obtained were suspended in a 10 mM Tris-HCl buffer solution (pH 7.4) to make a final concentration of 1.53 ml/g of tissue. The suspension was homogenized by a potter-type homogenizer for 5 strokes. The homogenate was stored at -80°C until the start of the experiment.

(Preparation of samples for measuring activity of serotonin 5-$HT_{1A}$ receptor)

Male Wistar rats were anesthetized with ether and were decapitated and the brains quickly extracted. The hippocampi were immediately collected from the brains. A 40-fold (vol/wt) amount of ice cooled 50 mM Tris-HCl buffer (pH 7.4) was added to the hippocampi. The mixture was homogenized using a potter-type homogenizer. The homogenate was centrifuged at 40,000 x g for 20 minutes. The supernatant liquid was removed and the resulting pellets were suspended in a 40-fold amount of Tris-HCl buffer solution. The suspension was centrifuged and the Tris buffer solution was again added to the pellets. This procedure was repeated twice. The product thus obtained was homogenized using a 40-fold amount of Tris-HCl buffer solution and incubated for 10 minutes at 37°C to remove endogenic 5-HT. The cell membrane was collected by centrifugation, washed twice, and suspended in a 10-fold amount of 50 mM Tris-HCl buffer solution (pH 7.4). The suspension thus obtained was stored at -80°C until the start of the binding experiment.

(Preparation of samples for measuring activity of cerotonin 5-$HT_{2A}$ receptor)

Male Wistar rats were anesthetized with ether and were decapitated. The brains were immediately extracted.

Cerebral membranes were collected from the brain. The brain tissue was immediately put into a 10-fold amount of ice cooled 0.25 M sucrose solution to prepare a homogenate using a potter-type homogenizer (4 strokes, 120 rpm). The homogenate was centrifuged for 10 minutes at 1,086 x g and the resulting pellets were again homogenized using the 0.25 M sucrose solution (1:5 w/v). The resulting homogenate was again centrifuged under the same conditions. The supernatant liquids were combined and diluted to a quantity of 40-fold with a Tris buffer solution. The suspension was centrifuged for 10 minutes at 35,000 x g. The resulting pellets were washed once by suspending the pellets using the same amount of Tris buffer solution, followed by centrifugation for 10 minutes at 35,000 x g. An appropriate amount of Tris buffer solution was added to the pellets to store the mixture at -80°C until the start of the experiment.

Results

Inhibitive concentration at 50% ($IC_{50}$) for each receptor of the vesamicol piperazine derivatives prepared in the Examples is shown in the following Table 5.

Table 5

| Compound | Vesamicol receptor | Adrenaline $\alpha_1$ receptor | Sigma $\alpha$-1 receptor | Serotonin receptor 5-$HT_{1A}$ | Serotonin receptor 5-$HT_{2A}$ |
|---|---|---|---|---|---|
| DRE348 | 0.78 | 55.2 | 1500 | N.D. | N.D. |
| DRE351 | 7.11 | 149 | 4500 | 664 | N.D. |
| DRE350 | 0.58 | 61.2 | 1500 | 240 | >20000 |
| DRE365 | 2.90 | 122 | N.D. | N.D. | N.D. |
| DRE366 | 1.14 | 53.6 | 203 | N.D. | N.D. |
| DRE334 | 0.72 | 21.8 | 109 | N.D. | N.D. |
| DRE335 | 2.80 | 16.4 | N.D. | N.D. | N.D. |
| DRE342 | 0.23 | 20.9 | 50 | 512 | N.D. |
| DRC140 | 2.12 | 402 | 8600 | 1340 | 3900 |
| DRE378 | 5.09 | N.D. | N.D. | N.D. | N.D. |
| DRE328 | 38.4 | N.D. | N.D. | N.D. | N.D. |
| DRF380 | 3.69 | N.D. | N.D. | N.D. | N.D. |
| DRF381 | 13.9 | 75.9 | N.D. | N.D. | N.D. |
| DRE353 | 8.74 | 147 | 6900 | N.D. | N.D. |
| DRE354 | 1.41 | 139 | 428 | N.D. | N.D. |
| DRF382 | 5.04 | 69.9 | 1300 | N.D. | N.D. |
| DRF383 | 8.74 | 147 | 6900 | N.D. | N.D. |

Example 32

Preparation of radioactive iodine-labeled

(-)-trans-5-tributyltin-2-hydroxy-3-[4-[2,5-dimethylphenyl] piperazinyl]tetralin

(-)-trans-5-tributyltin-2-hydroxy-3-[4-[2,5-dimethylphenyl]piperazinyl]tetralin (hereinafter referred to as "DRE363") obtained in Example 3 was labeled with radioactive iodine. 50 μl of DRE363 (20 mg/ml), 50 μl of 0.4 M phosphate buffer solution (pH 1.3), and 120 μl (98 mCi) of Na[$^{123}$I] were put into a glass tube (12ϕ x 75 mm), and 20 μl (10 mg/ml) of an oxidant, chloramine T, was added. The mixture was reacted for 1-1.5 minutes at room temperature. The labeling reaction was terminated by the addition of 20 μl of sodium hydrogensulfite (100 mg/ml), and the reaction solution was neutralized by the addition of 1 ml of saturated sodium hydrogencarbonate solution. Next, free I-123 was removed from the neutralized reaction solution using a cartridge for solid phase extraction (Waters, Sep-Pak Light C-18). After removal of the free 1-123, the labeled compound trapped by the cartridge was extracted using methanol and the extracted compound was concentrated by evaporating the solvent. The labeled compound was collected by reverse phase HPLC (column: Shiseido Co., Ltd. UG-120, ϕ 10 x 250 mm, mobile phase:
methanol:water:triethylamine = 90:10:0.2, flow rate = 2.0 ml/minute)from the residue after concentration.

The collected HPLC fraction was evaporated. After concentration, ascorbic acid and benzyl alcohol were added, followed by the addition of a physiological saline solution to provide the target concentration of an injection preparation. The injection preparation was analyzed by TLC (1. Reverse phase, Watman KC18, developing solvent; methanol:water = 80:20; 2. Normal phase, MERCK silica gel, development solvents; ethyl acetate:2-propanol:28% aqueous ammonia = 15:5:1). The radiochemical purity was 98.4% in the reverse phase and 96.0% in the normal phase. The Rf value of the labeled compound was 0.037 in the reverse phase and 0.738 in the normal phase.

Example 33

Preparation of radioactive iodine-labeled

(-)-trans-5-tributyltin-2-hydroxy-3-(4-phenyl-1-piperazinyl)tetralin

(-)-trans-5-tributyltin-2-hydroxy-3-(4-phenyl-1-piperazinyl)tetralin obtained in Example 23 was labeled with radioactive iodine in the same manner as in Example 32, except that 50 ml of a tributyltin compound DRC136 (50 mg/l), which is a precursor of DRC140, was used, 40 ml of 0.64% peracetic acid was added as an oxidant, and the mixture was reacted for one minute at room temperature while stirring. The labeled compound was purified by reverse phase HPLC (column: Shiseido Co., Ltd. UG-120, ϕ 10 x 250 mm, mobile phase;
methanol:water:triethylamine = 85:15:0.2, flow rate = 0.8 ml/minute). The purity of the labeled compound determined by the HPLC analysis was 98% or more.

Example 34

Specificity test for radioactive iodine labeled DRC140

A specificity test was carried out using DRC140 which is supposed to have high specificity among the piperazine derivatives shown in Table 5 of Example 31. This compound was known to reach equilibrium in 3 hours at 37°C. Therefore, iodine was labeled according to the method of Example 33 and the specificity of the radioactive-iodine-labeled DRC140 was examined as follows.

(1) Separation of synaptic vesicles from rat brain

Synaptic vesicles were extracted and separated from the brain of a rat to examine binding of the test compound with the synaptic vesicles. Specifically, the rats were anesthetized decapitated. The brains were immediately extracted. A 10-fold amount of 0.32 M sucrose solution was added to the brain tissue remaining after removal of the cerebellum. A homogenate was prepared by a potter-type homogenizer for 12 strokes at 1,000 rpm while cooling with ice. The homogenate was centrifuged at 1,100 x g for 10 minutes and the resulting supernatant was further centrifuged at 9,200 x g for 15 minutes. The pellets (P2 fractions) were collected and, after the addition of the 0.32 M sucrose solution, centrifuged for 15 minutes at 10,500 x g, thereby obtaining synaptosome fractions as pellets. To extract synaptic vesicles which were present in the synaptosome, purified water was added to the synaptosome fractions and the mixture was incubated for 30 minutes, thereby destroying cell membranes. To separate the synaptic vesicles from the destroyed

cell membranes, the incubated mixture was centrifuged at 25,500 x g. The resulting supernatant liquid was further centrifuged at 45,000 x g for 2 hours. The pellets (crude synaptic vesicles) obtained were purified by the sucrose density-gradient centrifugation method. Specifically, a concentration gradient from 0.4 M to 1.2 M was prepared in a centrifugal tube and the sample was layered over the upper layer. The resulting mixture was centrifuged at 53, 500 x g for two hours to separate a 0.4M band as synaptic vesicles. The amount of acetylcholine transporter in each fraction was confirmed by the Western blot method using an anti-acetylcholine transporter antibody.

(2) Saturation experiment and inhibition experiment for each cell fraction

A binding experiment for the cell fractions obtained in (1) above was carried out to determine the dissociation constant (Kd) and the maximum binding amount (Bmax). Specifically, each fraction of P2, crude synaptic vesicles, and synaptic vesicles was added to a test tube made of glass to make the protein amount 120 μg, 90 μg, and 45 μg, respectively. I-125 labeled DRC140 was added to the test tubes at varied concentrations in the range of 0.03 to 1.3 nM. The affinity of the I-125 labeled DRC140 to other receptors or binding sites was determined by adding an inhibitor which is specific to each receptor and measuring the reduction of specific binding of the I-125 labeled DRC140. Incubation was carried out for 3 hours at 37°C. The non-specific bond was determined by adding 20 mM (-)-vesamicol.

Assuming that the specific binding of a radioactive ligand without the inhibitor is 100%, the concentration of the inhibitor, at which up to 50% of the specific binding of a radioactive ligand ($IC_{50}$) is inhibited, was calculated using the formula in Example 31. A diagram of Scatchard plots prepared from the data was used to determine Bmax (the horizontal axis section) and Kd (the slope) . The results are shown in the following Table 6.

Table 6

| Fraction | Bmax | Kd |
|---|---|---|
| | fmol/mg protein | (nM) |
| P2 | 187±58.3 | 0.41±0.11 |
| Crude synaptic vesicles | 821±78.6 | 0.34±0.03 |
| Purified synaptic vesicles | 1751±92.6 | 0.31±0.00 |
| (average value ± standard deviation) | | |

The Scatchard plots exhibited a straight line in all fractions. As a result of determination of the maximum binding (Bmax) from the horizontal axis section, the increase in the Bmax of synaptosome purified by the density gradient centrifugation method was found to be approximately nine times as high as that of the P2 fractions. From the fact that the Bmax increases as the degree of purification of synaptic vesicles increases and the Kd exhibits no significant change, the radioactive-iodine-labeled DRC140 was proven to bind with the synaptic vesicles.

The concentrations of inhibitors for receptors, enzymes, and transporters which can inhibit binding of radioactive-iodine-labeled DRC140 to P2 fractions up to 50% ($IC_{50}$) are listed in Table 7, and those inhibiting to purified synaptic vesicle fractions up to 50% ($IC_{50}$) are listed in Table 8.

Table 7

| Inhibitor | Binding site | $IC_{50}$ (nM) |
|---|---|---|
| | | Average ± S.D |
| (-)-DRC140 | Vesamicol receptor | 1.52±0.12 |
| (+)-DRC140 | | 70.3±13.2 |
| (±)-Aminobenzo vesamicol | Vesamicol receptor | 1.17±0.22 |
| (-)-Vesamicol | Vesamicol receptor | 58.2±17.3 |
| (+)-Vesamicol | | >5000 |
| DTG | Sigma receptor | 3822±930 |
| Haloperidol | Sigma receptor/$D_2$ receptor | 1176±30.3 |
| NAN-190 | Serotonin 5-$HT_{1A}$ receptor | >5000 |
| Ketanserin | 5-$HT_2$ receptor | 1108±187 |

Table 7 (continued)

| Inhibitor | Binding site | $IC_{50}$ (nM) |
|---|---|---|
| | | Average ± S.D |
| LY-278,584 | 5-$HT_3$ receptor | >5000 |
| QNB | Muscarine | >5000 |
| Physostigmine | Ascetylcholine esterase | >5000 |
| 5-Methylurapidil | $\alpha_1$-Adrenalin | >5000 |
| (-)-Propranolol | β-Adrenalin | >5000 |
| ICI204, 448 | κ-Opioid receptor | >5000 |
| GYKI52466 | AMPA-Kainic acid receptor | >5000 |
| 5,7-Kynurenic acid | Glycine receptor | >5000 |
| Reserpine | Monoamine transporter | >5000 |

Table 8

| Inhibitor | Binding site | IC50 (nM) |
|---|---|---|
| | | Average ± S.D |
| (-)-DRC140 | Vesamicol receptor | 1.39±0.378 |
| (±)-Aminobenzo vesamicol | Vesamicol receptor | 1.67±0.093 |
| (-)-Vesamicol | Vesamicol receptor | 45.4±3.59 |
| DTG | Sigma receptor | 4158±251 |
| Haloperidol | Sigma receptor/$D_2$ receptor | 977±46 |
| Ketanserin | 5-$HT_2$ receptor | 1204±43 |

As can be seen from these Tables, no inhibitors, except for (±)-aminobenzo vesamicol and (-)-vesamicol which are the chemicals bonding to vesamicol receptors, can exhibit a strong inhibitive activity. DTG, haloperidol, and ketanserin exhibit a weak inhibitive action on P2 fractions. Because these chemicals also exhibit almost the same degree of weak inhibitive action on purified synaptic vesicles, they are thought to exhibit a weak binding force with synaptic vesicles. Based on the above results, the radioactive-iodine-labeled DRC140 was proven to specifically bind with vesamicol receptors on acetylcholine transporters, which are specifically present in synaptic vesicles.

Example 35

Measurement of activity of purified vesamicol piperazine derivatives on each receptor

The vesamicol piperazine derivatives, of which the activities on various receptors have been measured in the above Example, were further purified by HPLC (column: Shiseido Co., Ltd. UG-120, φ 15 x 250 mm, mobile phase: acetonitrile:water:triethylamine = 75:15:0.2) to obtain high purity derivatives. The activities of the high purity derivatives were measured.

Specifically, to measure the degree of affinity of these derivatives to vesamicol receptors, the degree of inhibiting binding with radioactive-iodine-label DRC140 ($IC_{50}$) was measured by using P2 fractions. To measure the affinity of the vesamicol receptors, the I-125 labeled DRC140, prepared in Example 33, was added to P2 fractions prepared from a brain homogenate in the same manner as in Example 31. The vesamicol piperazine derivatives synthesized in the Examples were added at various concentrations, and the mixture was incubated for 3 hours at 37°C.

The inhibitive concentration at 50% ($IC_{50}$) for each receptor of high purity derivatives is shown in the following Table 9.

Table 9

| Compound | Receptor | | | | |
|---|---|---|---|---|---|
| | Vesamicol | Sigma-1 | Alpha-1 | 5-$HT_{1A}$ | 5-$HT_{2A}$ |
| DRE348 | 1.16 | 5566 | | | |
| DRE351 | 11.4 | | | | |
| DRE350 | 0.83 | 5546 | | | |
| DRE366 | 0.88 | 203 | | | |
| DRE334 | 0.74 | 4481 | | | |
| DRE342 | 0.56 | | | | |
| DRC140 | 1.52 | 3371 | | | |
| DRF380 | 2.47 | 16288 | | | |
| DRF381 | 14.7 | 4776 | | | |
| DRE353 | 1.10 | 5106 | | | |
| DRE354 | 1.67 | 4563 | | | |
| DRF382 | 6.94 | | | | |
| DRF383 | 8.47 | | | | |
| DRG449 | 0.55 | 348 | 118 | 4769 | |
| DRE341 | 1.96 | | | | |
| DRF400 | 1.09 | 28778 | 15.6 | | |
| DRF401 | 0.89 | | 13.1 | | |
| DRF402 | 0.63 | | 5.89 | | |
| DRF403 | 2.42 | 3027 | 5.11 | | |
| DRF419 | 0.30 | 4706 | 3.10 | | |
| DRG446 | 0.38 | 544 | 13.2 | | |
| DRG454 | 0.59 | | | | |
| DRG455 | 0.58 | | | | |
| (+)-IBVM | 0.50 | | | | |
| (-)-IBVM | 0.32 | 174 | 2.61 | 119 | >5000 |
| (+)-MIBT | 0.89 | 4.27 | 421 | 16617 | |
| (-)-Vesamicol | 45.4 | 66.5 | | >5000 | >5000 |

Example 36

Distribution of radioactive-iodine-labeled DRE350 in rat body

80 µCi radioactive-iodine-labeled DRE350, labeled in Example 32, was administered to a male Wistar rat from the caudal vein under vigilance. The accurate dose was determined by measuring the radioactivity of the syringe before and after the administration using a Capintec's digital dosimeter. The rats were anesthetized with ether after 5, 10, 20, 40, 60, 90, and 240 minutes. Their heads were amputated. The skull was immediately removed and the brain was divided into the frontal lobe, occipital lobe, hippocampus, corpus striatum, cerebellum, and other portions. The radioactivity of each portion was measured. The radioactivity of a standard iodide solution with a known radioactivity was measured by a γ-counter. The radioactivity of each portion of the brain was determined using the radioactivity of the standard solution. The results are shown in Figure 1.

Accumulation of the compound in the corpus striatum was confirmed to be highest of all brain portions, maintaining a constant value from immediately after the administration. This shows that the compound binds strongly with vesamicol receptors of the acetylcholine transporters abundantly existing in the corpus striatum. Although the concentrations in the cerebral membrane and hippocampus decrease over time, the accumulation is seen to gradually become constant. On the other hand, the concentration in the cerebellum, in which there are almost no cholinergic neurons, is lowest of all brain portions, indicating that the accumulation of the radioactive compound is affected by distribution of the cholinergic neurons.

Example 37

Autoradiography of radioactive-iodine-labeled DRE350

Distribution of radioactive-iodine-labeled DRE350 prepared in Example 32 in the brain of a rat was examined by autoradiography. The radioactive-iodine-labeled compound was administered to male Wistar rat from the caudal vein, 50 μCi each time. The animal's head was amputated after four hours. The skull was immediately removed and the brain was embedded together with OTC on dry ice. The freeze-dry tissue was cut using a cryostat to prepare specimens with a thickness of 20 μm.

To examine the specificity of vesamicol binding sites of radioactive-iodine-labeled DRE350, 100 μl of 0.5 mg/kg cold vesamicol was administered in the caudal vein five minutes before administration of the radioactive-iodine-labeled DRE350. The specimens were prepared in the same manner. The results of autoradiography using these specimens are shown in Figure 2.

As can be seen from the results shown in Table 2, accumulation of the radioactive-iodine-labeled DRE350 is highest in the corpus striatum, wherein the connector neurons of acetylcholine is most abundant. The cerebral membrane and hippocampus showed a medium degree of accumulation.
Accumulation of the radioactive-iodine-labeled DRE350 in the cerebellum, wherein acetylcholine nerves are anatomically said to be most scarce, was smallest.

In addition, prior administration of non-labeled vesamicol was confirmed to decrease the accumulation in the corpus striatum, cerebral membrane, and hippocampus. Thus, accumulation of radioactive-iodine-labeled DRE350 in the rat body has been demonstrated to be specific.

Effect of the Invention

The vesamicol piperazine compound (I) of the present invention exhibits high specificity and strong affinity to vesamicol receptors. When labeled with radioactive iodine, the compound is rapidly absorbed into the brain after administration and specifically binds with the vesamicol receptors existing on the acetylcholine transporters in the biological bodies.

The distribution of the vesamicol receptors coincides with the distribution of acetylcholine nerves. Therefore, acetylcholine transporters existing in the center and periphery can be detected by examining the distribution of the vesamicol piperazine derivative (I).

In addition, the distribution of acetylcholine nerves can be non-invasively measured by measuring γ-rays emitted from radioactive isotopes such as radioactive iodine using a single photoemission tomography. Therefore, loss of acetylcholine nerves which are characteristically seen in Alzheimer's disease, but could have been detected only in the dissected brain after death, can be detected in a living brain. The vesamicol piperazine derivative (I) is thus useful as a tracer for SPECT in the early diagnosis of Alzheimer's disease.

## Claims

1. A vesamicol piperazine derivative or a salt thereof of the following formula:

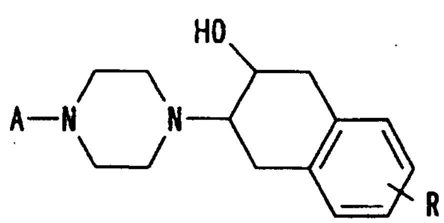

(I)

wherein R is a hydrogen atom, amino group, nitro group, non-radioactive or radioactive iodine atom, trialkyl tin group, or trial kyl silyl group, and A is a substituted or unsubstituted aryl group or a heterocyclic group.

2. The vesamicol piperazine derivative or the salt thereof according to claim 1, wherein the substituent for the group A is one or more groups selected from the group consisting of lower alkyl groups, amino groups, aminoalkyl groups, alkylamide groups, hydroxyl groups, hydroxyalkyl groups, lower alkoxy groups, non-radioactive or radioactive iodine atoms, trialkyltin groups, and trialkylsilyl groups.

3. The vesamicol piperazine derivative or the salt thereof according to claim 1, wherein the group A is a phenyl group, tolyl group, xylyl group, pyridyl group, or pyrimidyl group.

4. An agent comprising the vesamicol piperazine derivative or the salt thereof according to any one of claims 1 to 3.

5. The agent according to claim 4 used for measuring central or peripheral acetylcholine transporters.

6. An agent for measuring central or peripheral acetylcholine transporters according to claim 5 used with single photon emission computed tomography (SPECT).

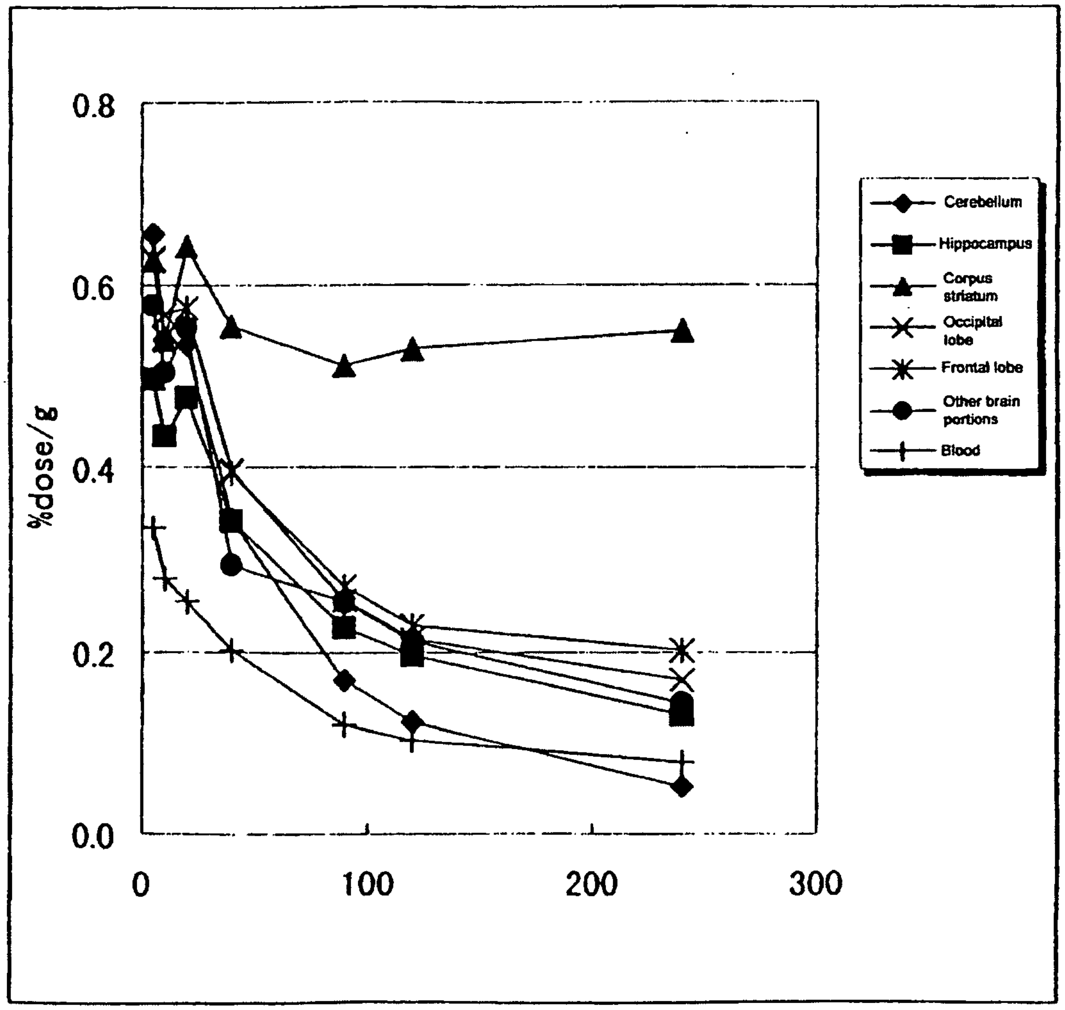
0.8
0.6
0.4
0.2
0.0
%dose/g
0
100
200
300
Cerebellum
Hippocampus
Corpus striatum
Occipital lobe
Frontal lobe
Other brain portions
Blood

*FIG. 1*

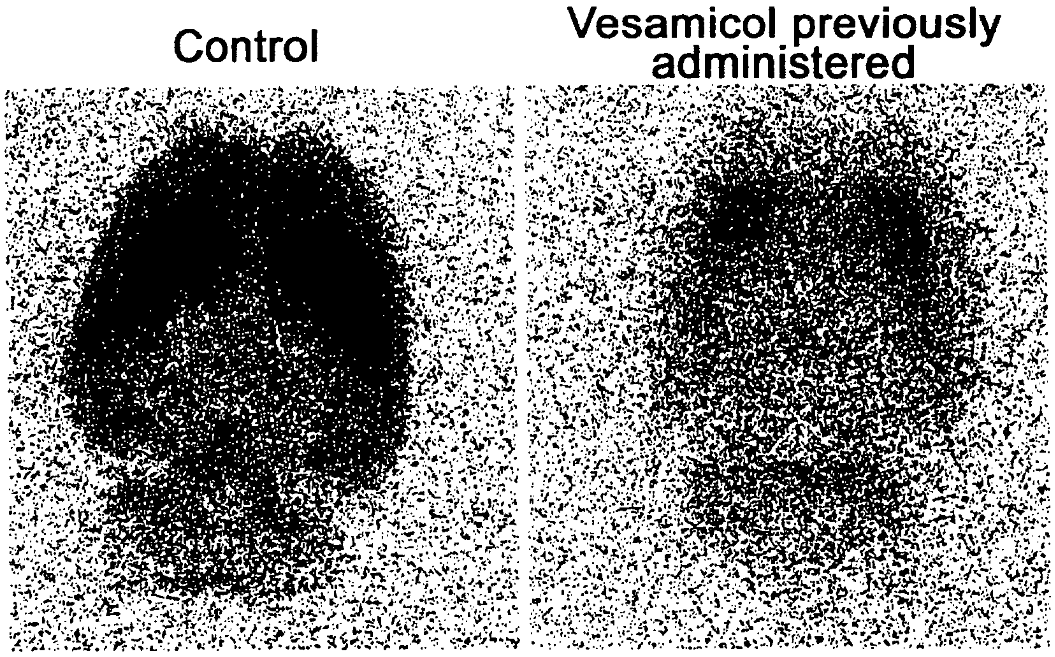


*FIG. 2*

## INTERNATIONAL SEARCH REPORT

International application No.
PCT/JP00/04950

A. CLASSIFICATION OF SUBJECT MATTER
Int.Cl$^7$ C07D295/096, C07D295/135, A61K31/495, A61K51/04

According to International Patent Classification (IPC) or to both national classification and IPC

B. FIELDS SEARCHED

Minimum documentation searched (classification system followed by classification symbols)
Int.Cl$^7$ C07D295/096, C07D295/135, A61K31/495, A61K51/04

Documentation searched other than minimum documentation to the extent that such documents are included in the fields searched

Electronic data base consulted during the international search (name of data base and, where practicable, search terms used)
REGISTRY(STN),CA(STN),CAOLD(STN),CAPLUS(STN)

C. DOCUMENTS CONSIDERED TO BE RELEVANT

| Category* | Citation of document, with indication, where appropriate, of the relevant passages | Relevant to claim No. |
|---|---|---|
| X<br>A | US, 4018773, A (E.R.Squibb & Sons,Inc.), 19 April, 1977 (19.04.77), Full text; especially, see examples 2, 3 & JP, 52-036674, A & DE, 2641499, A & GB, 1564527, A & FR, 2323388, A | 1-4<br>5,6 |
| X<br>A | IVANOV, I. et al., "Derivatives of 2-amino-1,2,3,4-tetrahydron aphthalene, III. Synthesis of some N'-substituted N-(trans-3-hydroxy-1,2,3,4-tetrahydro-2-naphthyl)piperazines", Arch. Pharm. (Weinheim, Ger.) (1977), Vol.310, No.11, p.925-31 | 1,3<br>2,4-6 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 October, 2000 (17.10.00) | 31 October, 2000 (31.10.00) |
| Name and mailing address of the ISA/<br>Japanese Patent Office | Authorized officer |
| Facsimile No. | Telephone No. |

Form PCT/ISA/210 (second sheet) (July 1992)